(19) Europäisches Patentamt / European Patent Office / Office européen des brevets

(11) **EP 2 637 559 B1**

(12) **EUROPEAN PATENT SPECIFICATION**

(45) Date of publication and mention
of the grant of the patent:
**24.01.2018 Bulletin 2018/04**

(51) Int Cl.:
*A61B 5/145* (2006.01)   *A61B 5/1455* (2006.01)
*A61B 5/026* (2006.01)   *A61B 5/00* (2006.01)
*A61B 5/02* (2006.01)

(21) Application number: **10859470.6**

(22) Date of filing: **10.11.2010**

(86) International application number:
**PCT/US2010/056282**

(87) International publication number:
**WO 2012/064326 (18.05.2012 Gazette 2012/20)**

(54) **OPTICAL MEASUREMENT OF PARAMETERS RELATED TO MOTION OF LIGHT-SCATTERING PARTICLES WITHIN A FLUID BY MANIPULATING ANALOG ELECTRICAL SIGNALS**

OPTISCHE MESSUNG VON PARAMETERN IN BEZUG AUF DIE BEWEGUNG VON LICHTSTREUENDEN PARTIKELN IN EINER FLÜSSIGKEIT DURCH MANIPULIEREN ANALOGER ELEKTRISCHER SIGNALE

MESURE OPTIQUE DE PARAMÈTRES ASSOCIÉS AU MOUVEMENT DE PARTICULES DE DIFFUSION DE LUMIÈRE DANS UN FLUIDE PAR MANIPULATION DE SIGNAUX ÉLECTRIQUES ANALOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**18.09.2013 Bulletin 2013/38**

(73) Proprietor: **Elfi-Tech Ltd.**
**76705 Rehovot (IL)**

(72) Inventors:
• **FINE, Ilya**
**76302 Rehovot (IL)**
• **KAMINSKY, Alexander**
**76341 Hod Hasharon (IL)**

(74) Representative: **Harrison IP Limited**
**3 Ebor House**
**Millfield Lane**
**Nether Poppleton, York YO26 6QY (GB)**

(56) References cited:
WO-A2-2008/053474   US-A- 4 476 875
US-A- 5 218 962   US-A1- 2003 069 487
US-A1- 2004 024 297   US-A1- 2009 209 834

• **MACFADYEN A J ET AL: "Fibre-optic systems for dynamic light scattering - a review",** OPTICS AND LASER TECHNOLOGY, ELSEVIER SCIENCE PUBLISHERS BV., AMSTERDAM, NL, vol. 22, no. 3, 1 June 1990 (1990-06-01), pages 175-187, XP024436344, ISSN: 0030-3992, DOI: 10.1016/0030-3992(90)90105-D [retrieved on 1990-06-01]
• **KALCHENKO V ET AL: "In vivo dynamic light scattering imaging of blood coagulation",** JOURNAL OF BIOMEDICAL OPTICS, S P I E - INTERNATIONAL SOCIETY FOR OPTICAL ENGINEERING, US, vol. 12, no. 5, 12 September 2007 (2007-09-12), pages 52002-1, XP002551829, ISSN: 1083-3668, DOI: 10.1117/1.2778695 [retrieved on 2007-09-12]

**Description**

**FIELD OF THE INVENTION**

The present invention relates to a method for optical measurement of a parameter related to motion of light-scattering particles within a fluid by manipulating analog electrical signals.

**BACKGROUND AND RELATED ART**

Near infrared spectroscopy (NIRS) is a well-established non-invasive technique which allows for the determination of tissue and blood analytes conditions based on spectro-photometric measurements in the visible and near-infrared regions of the spectrum of light. According to this technique, incident light penetrates the examined skin, and reflected and/or transmitted light is/are measured. In order to quantify any blood analyte, light of at least two different wavelengths is required. Optical plethysmography, pulse oximetry, and occlusion spectroscopy are the most prominent examples of usage of the NIR spectroscopy in medicine and physiological studies.

Visible or near infrared light is commonly used to track the optical manifestation of some time-dependent physiological processes. Such prolonged measurement of light response as a function of time can provide clinician with valuable information about time-dependent physiological processes.

For example, the measured light response of a natural heart beat pulsation is varied with each pulse. The signal is then measured at one point of the pulse wave and compared with the signal at another point. The difference between the values is due to arterial blood alone. In the pulse-oximetry, this phenomenon is utilized for the determination of oxy-hemoglobin saturation.

In the case of occlusion spectroscopy, the optical time-dependent signal is originated by light scattering changes associated with the red blood cells (RBC) aggregation process. In this case, the optical signal changes are utilized for the hemoglobin or glucose measurement.

Yet another known method enables to generate the required changes is the application of a periodic or non-periodic local pressure variation, resulting in blood volume fluctuations. These fluctuations are used to measure different blood parameters, like hemoglobin or glucose.

The major underlying assumption in the processing of all kind of the time-dependent signals is that the measured optical variation is originated solely by blood related components. In pulse oximetry, for example, it is commonly accepted that arterial blood volume changes are the only responsible factor staying behind the optical signal modulation. However, a more complex physical analysis shows that even if the only changes in the system are ascribed to the blood, the measured optical response of these changes is a convolution of absorption and scattering properties of blood and surrounding media. While carrying out any algorithmic modeling and signal processing procedure of these measured optical signals, the tissue related effects can not be disregarded. Therefore, the common denominator of all time-dependent signal related optical methods relies on the measurement of optical responses originated by the blood dynamics or hemorheological status changes.

It should be noted that the accuracy of time-dependent methods depends on the ability to identify the hemorheological component of the blood. For example, in the particular case of pulse-oximetry, the heart beats modulate the hemorheological status of circulating blood, resulting in the fluctuation of RBC velocity, which is associated with the shear forces changes. The variation of the hemorheological blood parameters enables to optically distinguish the pulse-related changes of the signal. Therefore, the decreased accuracy in the determination of hemorheological properties leads to a lower accuracy in the determination of the sought blood parameter. Among the blood parameters which can be derived from the hemorheological changes are hemoglobin oxygen saturation, carohyhemoglobin (percentage of HbCO out of total hemoglobin), hemoglobin blood concentration and/or glucose.

Moreover, the arterial blood pressure is another physiological parameter, which is commonly derived from the hemorheological related variations. The systolic blood pressure can be determined with assistance of inflating cuff which induces hemorheological variations artificially. When a pressure beyond the systolic pressure is applied, no pulsatile waveform appears at the down- flow. The diastolic point of the pressure is frequently measured by using Korotkoffs sounds. The source of these sounds is associated with abrupt changes in hemorheological properties of blood, occurring due to deflation of cuff from the systolic point. These hemorheological changes, in the vicinity of the diastolic point, result in a very typical pattern of sound, which can be detected by a stethoscope or by other acoustic device. However, the sound related method is very sensitive to different motion artifacts and therefore in automatic blood pressure devices, commonly used for the self-monitoring, the accuracy of blood pressure reading is impaired.

The following published patent documents provide potentially relevant background art, WO 2007/144880, WO 2007/113804, WO 2008/053474 and United States patent 4,476,865.

The following non-patent publications provide potentially relevant background art: (i) Macfadyen, A. J., and B. R. Jennings. "Fibre-optic systems for dynamic light scattering-a review." Optics & Laser Technology 22.3 (1990): 175-187;

and (ii) Kalchenko, Vyacheslav, et al. "In vivo dynamic light scattering imaging of blood coagulation." Journal of biomedical optics 12.5 (2007): 052002-052002.

A method according to the preamble of claim 1 is known from WO 2008/053473 A2.

## SUMMARY OF EMBODIMENTS

Embodiments of the present invention relate to a method for *in vitro* or *in vivo* optical measurement of a parameter related to motion of light-scattering particles within a fluid by manipulating analog electrical signals. In some preferable embodiments, at least partially coherent light (e.g. NIR light) is employed.

Embodiments of the present invention relate to dynamic light scattering (DLS) measurements where information about motion of 'small' particles (e.g. micron sized or sub-micron particles such as red blood cells or biological macromolecules or colloids) is derived from time fluctuations of a coherent light interference pattern. In different embodiments, it is possible to derive or measure any combination of: rheological parameter(s), physiological parameter(s), blood clotting parameter(s), a blood pressure or pulse rate, or other physical or chemical or mechanical parameter(s) relating to motion of a particle (i.e. biological or non-biological particle) within a fluid (i.e. a biological fluid such as blood plasma or a non-biological fluid such as a petroleum-related fluid or any other fluid).

Using first and second photodetectors respectively located in first and second locations, it is possible to generate first and second analog electrical signals from a location-dependent light field that is influenced by a light-response signal caused by scattering of the particles within the fluid. The first and second analog electrical signals respectively describe the location-dependent light field in the first and second locations. In many or most or all cases, for each of the first and second analog electrical signals, the analog electrical signal respectively is dominated by factors other than the time-fluctuating light interference patterns observable by scattering of light from particles within the fluid.

However, by manipulating these two analog electrical signals (i.e. the 'first' and 'second' analog electrical signals), it is possible to generate a third or 'hybrid' analog electrical signal (e.g. a 'difference' electrical signal describing a difference between the first and second analog electrical signals). For this third electrical signal, the fractional contribution, to overall analog electrical signal power, due to the aforementioned time-fluctuating light interference patterns caused by scattering of light off of particles, is much greater than what can be observed for the first and second analog electrical signals. In this sense, it is possible to first 'boost' the relative signal contribution due to light scattering off of particles, and then obtain measurement parameters form the analog electrical signal having the boosted relative contributions.

Thus, in some embodiments, even though the input analog electrical signals (i.e. derived from light field measurements at the first and second locations) may be closely correlated with each other (e.g. because the first and second locations are relatively 'close' to each other), the relatively small components of each analog signal attributable to time-fluctuating light interference patterns may be substantially uncorrelated with each other.

By generating the third analog signal (e.g. by subtracting one signal from the other), it is possible to obtain an analog signal (i) whose overall power level is much weaker than the power level of the input analog electrical signals (i.e. because generating a difference between two substantially equal electrical signals comes close to 'cancelling out' the overall signal), but (ii) whose fractional component attributable to time-fluctuating light interference patterns is much greater than what may be observed in either the first or second analog electrical signals.

In some embodiments, because the third analog signal is partially or mostly or completely 'purged' of components that are not related to scattering-derived fluctuations of coherent light interference pattern, it possible to amplify the resultant 'third' analog electrical signal without overwhelming any 'signal analysis circuitry' with electrical signal components that are unrelated to light scattering off of the particles and/or irrelevant for deriving the motion-related parameters of the particles within the fluid.

Examples of such 'irrelevant' components whose importance may be reduced include .light-derived electrical signals due to motion artifacts, due to ambient light and/or due to regularly-behaved fluctuations in the analog electrical signal (e.g. light fluctuations of a fluorescent light bulb operating at 50 HZ or 60 HZ).

A method for obtaining physiological information about a subject comprises: a) illuminating a portion of the subject's skin to scatter partially or entirely coherent light off of moving red blood cells (RBCs) within the subject's blood to influence a location-dependent light field; b) detecting light of the location-dependent light field at first and second locations to respectively generate first and second analog electrical signals that respectively describe the light field at the first and second locations; c) generating a difference analog electrical signal that describes a difference between the first and second analog signals; d) analyzing the difference analog electrical signal or a derivative thereof to characterize time fluctuations of a coherent light interference pattern described by the difference analog signal; and e) deriving, according to results of the analysis, at least one physiological parameter of the subject.

In some embodiments, the difference analog signal generating is carried out so that a ratio between: i) a magnitude of electrical power of the difference analog signal; and ii) an average between a magnitude of electrical power of the first electrical signal and a magnitude of electrical power of the second electrical signal, is at most 0.2 or at most 0.1 or at most 0.05 or at most 0.025 or at most 0.01 or at most 0.005 or at most 0.001.

In some embodiments, the physiological parameter is a blood coagulation parameter and/or a property of blood rheology and/or an in vivo heart pulse rate and/or a systolic and/or diastolic arterial blood pressure value and/or a concentration of a micron or sub-micron particle.

In some embodiments, the physiological parameter is a concentration of a micron or sub-micron biological particle such as a red blood cell or a biological macromolecule (e.g. a protein or any other biological macromolecule).

In some embodiments, the physiological parameter is a diffusion coefficient of a biological particle within a biological fluid, a biological particle size, or a biological particle mobility (i.e. a Stokes-Einstein mobility that may or may not exactly follow the Stokes-Einstein law).

In some embodiments, analyzed derivative thereof (i.e. derivative of the difference analog signal) is a digital signal generated by amplifying the difference analog signal by at least a factor of 10 (or at least a factor of 5, or at least a factor of 20 or at least a factor of 50 or at least a factor of 100) and digitizing the amplified signal.

In some embodiments, the analysis is carried out by any combination of analog electronic circuitry, digital electronic circuitry, and software.

There is no explicit limitation on an offset distance between the first and second locations. In some embodiments , this offset distance is at least 10 or at least 50 or at least 100 or a least 250 microns or at least 500 microns and/or at most 10 cm or at most 5 cm or at most 3 cm or at most 2 cm or at most 1 cm.

In some embodiments, the detecting at the first and second locations is carried out respectively by first and second photodetectors.

In some embodiments, a ratio between: i) a stochastic power fraction of the difference analog electrical signal; and ii) an average between stochastic power fractions of the first and second analog electrical signals is at least 10 (or at least 5 or at least 20 or at least 50 or at least 100).

Apparatus for obtaining physiological information about a subject, the apparatus comprises: a) one or more sources of partially or entirely coherent light configured to illuminate a portion of the subject's skin to scatter partially or entirely coherent light off of moving red blood cells (RBCs) within the subject's blood to influence a location-dependent light field; b) a plurality of photodetectors including first and second photodetectors respectively configured to detect light of the location-dependent light field at first and second locations to respectively generate first and second analog electrical signals that respectively describe the light field at the first and second locations; c) electronic circuitry including analog circuitry that is configured to generate a difference analog electrical signal that describes a difference between the first and second analog signals, the electronic circuitry further configured to: i) analyze the difference analog electrical signal or a derivative thereof to: characterize time fluctuations of a coherent light interference pattern described by the difference analog signal; and ii) derive, according to results of the analysis, at least one physiological parameter of the subject.

A method for obtaining physiological information about a subject comprises a) illuminating a portion of the subject's skin to scatter partially or entirely coherent light off of moving red blood cells (RBCs) within the subject's blood to influence a location-dependent light field; b) detecting light of the location-dependent light field at first and second locations to respectively generate first and second analog electrical signals that respectively describe the light field at the first and second locations; c) generating a hybrid analog electrical signal from the first and second analog electrical signals such that a ratio between: i) a stochastic power fraction of the difference analog electrical signal; and ii) an average between stochastic power fractions of the first and second analog electrical signals is at least 10 (or at least 5 or at least 20 or at least 50 or at least 100) d) analyzing the hybrid analog electrical signal or a derivative thereof to characterize time fluctuations of coherent light interference pattern described by the hybrid analog signal; and e) deriving, according to results of the analysis, at least one physiological parameter of the subject.

Apparatus for obtaining physiological information about a subject, the apparatus comprising: a) one or more sources of partially or entirely coherent light configured to illuminate a portion of the subject's skin to scatter partially or entirely coherent light off of moving red blood cells (RBCs) within the subject's blood to influence a location-dependent light field; b) a plurality of photodetectors including first and second photodetectors respectively configured to detect light of the location-dependent light field at first and second locations to respectively generate first and second analog electrical signals that respectively describe the light field at the first and second locations; c) electronic circuitry including analog circuitry that is configured to generate a hybrid analog electrical signal from the first and second analog electrical signals such that a ratio between: i) a stochastic power fraction of the difference analog electrical signal; and ii) an average between stochastic power fractions of the first and second analog electrical signals is at least 10 (or at least 5 or at least 20 or at least 50 or at least 100). , the electronic circuitry further configured to A) analyze the difference analog electrical signal or a derivative thereof to characterize time fluctuations of a coherent light interference pattern described by the difference analog signal; and B) derive, according to results of the analysis, at least one physiological parameter of the subject.

An apparatus for obtaining information related to the movement of particles within a fluid, the apparatus comprising: a) one or more light sources configured to induce a light response signal by applying partially or entirely coherent light so that at least some of the light is scattered by the moving particles within the fluid, the light response signal contributing to a location-dependent light field; b) a detection system including first and second photodetectors respectively located at first and second locations, the photodetectors respectively configured to respectively generate, from the light field at the first and second locations, first and second photodetector analog electrical signals; c) electronic circuitry including analog circuitry that is configured to generate, from the first and second photodetector analog electrical signals, a difference analog electrical signal describing a difference between the light field at the first location and the light field at the second location, the electronic circuitry being configured, when a power level of the difference analog electrical signal is significantly less than a power level of the first and second analog electrical signals to i) analyze the analog difference signal or a derivative thereof to characterize time fluctuations of a coherent light interference pattern described by the analog difference signal; and ii) deriving, from results of the analysis, at least one of: A) information describing the motion of the particles within the fluid; B) a physiological or biological parameter related to motion of the particles within the fluid.

In some embodiments, the electronic circuitry includes digital circuitry and/or software, the electronic circuitry being configured such that the deriving of the motion-describing information or of the physiological or biological parameter is carried out, at least in part, by the digital circuitry and/or software.

In some embodiments, the deriving of the motion-describing information or of the physiological or biological parameter is carried out, at least in part, by the digital circuitry and/or software, by analog circuitry.

In some embodiments, the electronic circuitry is further operative to: amplify, by a factor of at least 10 (or at least 5 or at least 20 or at least 50 or at least 100)., the difference analog signal to obtain an amplified difference analog signal; and digitize the amplified difference analog signal to obtain a digitized signal, and wherein the analyzing and deriving is performed digitally using the digitized signal.

In some embodiments, the motion-related information is selected from the group consisting of: i) a diffusion coefficient of particles within the fluid; ii) a mobility coefficient (i.e. related in some way to the Stokes-Einstein law) of particles within the fluid; iii) a viscosity of the fluid or of a suspension including the fluid and the particles; iv) a rheological property of the fluid or of a suspension including the fluid and the particles; v) a particle size or shape or flexibility; vi) a micron or sub-micron particle concentration; vii) an intensity of particle Brownian motion; and viii) a particle mean-free path within the fluid.

In some embodiments, the electronic circuitry is operative to derive in-vivo biological information from a biological fluid within or on a subject.

In some embodiments, the biological fluid is selected from the group consisting of blood, sweat and lymph fluid.

In some embodiments, the electronic circuitry is operative to derive biological information from an in-vitro biological fluid.

In some embodiments, the electronic circuitry is operative to derive at least one of: i) a blood rheological parameter (e.g. blood viscosity); ii) a blood particle size; iii) a blood coagulation rate; iv) a blood viscosity; v) a pulse parameter; vi) a blood pressure parameter; vii) a red blood cell (RBC) aggregation parameter; viii) an RBC velocity.

In some embodiments, the electronic circuitry is configured to effect the analysis and the information or parameter deriving when a power level of the difference analog electrical signal is at most a fraction of an average (or minimum or maximum) power level of the first and second analog signals, a value of the fraction being at most 0.1. (or at most 1/5 or at most 1/20 or at most 1/50 or at most 1/100 or at most 1/1000).

In some embodiments, the photodetectors are mounted to a substrate or embedded in or etched into a substrate.

In some embodiments, the apparatus further comprises: d) a pressurizing assembly operative to induce a change in blood flow by applying a pressure or a force of a subject's skin.

In some embodiments, the pressurizing assembly includes: i) a strap for constricting blood flow in a subject's finger; and ii) a pneumatic tube for tightening the strap around the subject's finger to constrict the blood flow.

In some embodiments, the pressurizing assembly is operative to create an intermittent blood stasis state by applying over systolic blood pressure to the subject.

In some embodiments, the digital processing unit is operative to effect at least one of (i) a temporal autocorrelation analysis of the digitized signal; (ii) effecting a power spectrum analysis of the digitized signal; and wherein the particle-describing motion information and/or the biological or physiological parameter is derived in accordance with results of the temporal autocorrelation and/or power spectrum analysis.

An apparatus for obtaining information related to the movement of particles within a fluid comprises: a) one or more light sources configured to induce a light response signal by applying partially or entirely coherent light, the light response signal contributing to a location-dependent light field; b) a detection system including first and second photo-detectors respectively located at first and second locations, the photodetectors respectively configured to respectively generate, from the light field at the first and second locations, first and second photodetector analog electrical signals; c) electronic circuitry including analog circuitry that is configured to generate, from the first and second photodetector analog electrical signals, a difference analog electrical signal describing a difference between the light field at the first location and the light field at the second location, the electronic circuitry being configured to: i) analyze the analog difference signal or a derivative thereof to characterize time fluctuations of a coherent light interference pattern described by the analog difference signal; and ii) deriving, from results of the analysis, at least one of: A) information describing the motion of the particles within the fluid; B) a physiological or biological parameter related to motion of the particles within the fluid.

An apparatus for obtaining information related to the movement of particles within a fluid comprises: a) one or more light sources configured to induce a light response signal by applying partially or entirely coherent light, the light response signal contributing to a location-dependent light field; b) a detection system including first and second photo-detectors respectively located at first and second locations, the photodetectors respectively configured to respectively generate, from the light field at the first and second locations, first and second photodetector analog electrical signals, each analog electrical signal respectively including stochastic and non-stochastic components and respectively associated with a stochastic fraction describing relative power levels of the stochastic and non-stochastic components; c) electronic circuitry including analog circuitry that is configured to process the first and second analog signals so as to obtain a hybrid analog signal, a stochastic fraction of the hybrid analog signal exceeding stochastic fractions of both the first and second analog electrical signals by a factor of at least 10 (or at least 5 or at least 20 or at least 50 or at least 100).; the electronic circuitry being configured to: i) analyze the analog difference signal or a derivative thereof to characterize time fluctuations of a coherent light interference pattern described by the hybrid analog signal; and ii) deriving, from results of the analysis, at least one of: A) information describing the motion of the particles within the fluid; B) a physiological or biological parameter related to motion of the particles within the fluid.

An apparatus for obtaining information related to the movement of particles within a fluid comprises: a) one or more light sources configured to induce a light response signal by applying partially or entirely coherent light, the light response signal contributing to a location-dependent light field; b) a detection system including first and second photo-detectors respectively located at first and second locations, the photodetectors respectively configured to respectively generate, from the light field at the first and second locations, first and second photodetector analog electrical signals, each analog electrical signal respectively including correlated and non-correlated components describing a fraction of one of the first and second analog signals that is correlated with the other of the first and second analog signals, a correlation fraction describing relative power levels of the correlated and non-correlated components; c) electronic circuitry including analog circuitry that is configured to process the first and second analog signals so as to obtain a hybrid analog signal having first and second correlated component describing correlation with the first and second analog signals, a non-correlation fraction of the hybrid analog signal exceeding correlations fractions of both the first and second analog electrical signals by a factor of at least 10 (or at least 5 or at least 20 or at least 50 or at least 100).;, the electronic circuitry being configured to: i) analyze the analog difference signal or a derivative thereof to characterize time fluctuations of a coherent light interference pattern described by the hybrid analog signal; and ii) deriving, from results of the analysis, at least one of: A) information describing the motion of the particles within the fluid; B) a physiological or biological parameter related to motion of the particles within the fluid.

A method for obtaining information related to the movement of particles within a fluid comprises: a) scattering partially or entirely coherent light off of particles within a fluid to influence a location-dependent light field; b) detecting the location-dependent field at two different locations to respectively generate a first analog signal describing the light field at the first location and a second analog signal describing the light field at the second location; c) generating a difference analog signal that describes a difference between the first and second analog signals; d) analyzing the difference analog signal to obtain characterize time fluctuations of a coherent light interference pattern described by the difference analog signal; e) deriving, from results of the analysis, at least one of: i) information describing the motion of the particles within the fluid; ii) a physiological or biological parameter related to motion of the particles within the fluid.

An apparatus for obtaining physiological information from a subject the apparatus comprises: a) one or more light sources configured to apply partially or entirely coherent light to a target region of the subject to induce a light response signal from the illuminated target region; b) a detection system including: a) a first photodetector configured to detect the light response signal prevailing at a first location to generate a first photodetector analog electrical signal; b) a second photodetector configured to detect the second light response signal prevailing at a second location to generate a second photodetector analog electrical signal; c) an analog electronics assembly configured to generate, from the first and second analog electrical signals, a difference analog electrical signal that is indicative of a difference between the light response signal at the first location and at the second location (in some embodiments, the difference analog signal is indicative of a difference between a light field prevailing at the first location and a light field prevailing at the second location); d) a digitizer (i.e. A to D converter) for digitizing the difference analog electrical signal to generate a digitized signal; and e) a digital processing unit operative to receive and analyze the digitized signal to compute at least one physiological parameter of the subject.

In some embodiments, the second location is offset from the first location by an offset distance that is less than 5 cm or less than 2 cm or less than 1 cm or less than 5 mm or less than 2.5 mm or less than 1 mm or less than 0.5 mm and/or at least 0.01 mm or at least 0.05 mm or at least 0.1 mm or at least 0.25 mm.

In some embodiments, the apparatus further comprises: g) a pressurizing assembly operative to induce a change in blood flow at or near the target region by applying a pressure or a force to the subject's skin.

In some embodiments, this may be useful for determining a blood rheological parameter. For example, this may be useful for determining systolic blood pressure to thereby determine RBC aggregation - for example, a rate of aggregation of an average particle size - the skilled artisan is directed to WO 2008/053474 for details.

In some embodiments, the pressurizing assembly includes: i) a strap for constricting blood flow in a subject's finger; and ii) a pneumatic tube for tightening the strap around the subject's finger to constrict the blood flow. In some embodiments, the pressurizing assembly is operative to create an intermittent blood stasis state by applying over systolic blood pressure to the subject, thereby enabling determination of red blood cell (RBC) aggregation.

In some embodiments, the digital processing unit is configured: (i) to detect stochastic time-dependent fluctuations in a magnitude of the difference analog electrical signal; and (ii) to compute the at least one physiological parameters in accordance with the detected stochastic fluctuations.

In some embodiments, the digital processing unit is operative to compute a blood velocity and/or blood rheology parameter from the digitized signal.

In some embodiments, the digital processing unit is operative to compute from the digitized signal at least one parameter selected from the group consisting of a blood viscosity, a blood particle size (for example, a central tendency such as an average of RBC aggregates) and a blood coagulation rate.

In some embodiments, the first photodetector is situated at the first location and the second photodetector is situated at the second location, and a distance between the first and second detectors is the offset distance.

In some embodiments, i) the first and/or second photodetectors are respectively situated away (for example, separated by at least 1 cm or 2 cm or 5 cm or more) from the first and second locations and respectively receive light respectively from the first and/or second location via an optical fiber.

In some embodiments, the digital processing unit is operative to effect at least one of (i) a temporal autocorrelation analysis of the digitized signal; (ii) effecting a power spectrum analysis of the digitized signal, wherein the at least one physiological parameter is computed in accordance with results of the temporal autocorrelation and/or power spectrum analysis.

In some embodiments, the analog electronics assembly: i) is configured to receive: A) a first input analog signal derived from the first analog photodetector analog electrical signal; and B) a second input analog signal derived from the second analog photodetector analog electrical signal; and ii) to generate the difference analog electrical signal from a difference between the first and second input signals.

In some embodiments, the one or more light sources are part of an "illuminating system" which may include one or more optical components for example, for focusing the coherent light.

There is no limitation on the wavelength of coherent light that may be used. In some embodiments, the coherent light may include wavelengths between 350 nm and 2,000 nm, for example, visible (for example, red) and/or near infrared (NIR) light.

In one non-limiting example, the coherent light is red and/or NIR light which may be useful for determining a light of read and blood oxygen saturation and/or a blood hemoglobin concentration.

There is no limitation on the type of photodetector that may be used. In one non-limiting example, the first and/or second photodetector is a Charge-coupled devices (CCD).

A method for obtaining physiological information from a subject in accordance with a light field whose intensity varies according to location is now disclosed. The method comprises: a) applying partially or entirely coherent light to a target region of the subject to induce a light response signal from the illuminated target region which contributes to a location-dependent light field; b) detecting a light field signal prevailing at a first location to generate a first analog signal, the first analog signal including a DLS component that is indicative of a first dynamic light scattering (DLS) measurement; c) detecting a light field signal prevailing at a second location to generate a second analog signal including a DLS component that is indicative of a second dynamic light scattering (DLS) measurement a ratio and that is uncorrelated to the DLS component of the first analog signal; d) processing the first and second analog signals to generate a processed analog signal including a DLS component that is indicative of a combination of the first and second dynamic scattering measurements wherein a ratio between: i) a boosted DLS contribution ratio between a magnitude of the DLS component of the processed analog signal and the magnitude of the processed analog signal; and ii) at least one of a first DLS contribution ratio and a second DLS contribution ratio, is at least 10 and wherein: i) the first DLS contribution ratio is defined as a ratio between a magnitude of the DLS component of the first analog signal and the magnitude of the first analog signal; and ii) the second output ratio is defined as a ratio between a magnitude of the DLS component of the second analog signal and the magnitude of the second analog signal.

In some embodiments, the method further comprises: e) digitizing the processed analog signal to generate a digital signal indicative of a combination of the first and second dynamic scattering measurements; and f) processing the indicative digital signal to compute at least one physiological parameter.

In some embodiments, the computing includes computing at least one of a blood velocity, a blood rheological parameter, a blood pressure, a heartbeat, a blood oxygen concentration, and a pulse.

In some embodiments, the method further comprises: e) before the light field detecting, inducing a change in blood flow at or near the target region.

In some embodiments, the blood flow change inducing is carried out by applying a force or a pressure to the subject.

In some embodiments, the target region is located in a location selected from the group consisting of a finger region, a back region, a leg region, a face region and an arm region.

In some embodiments, the method is carried out so that the first output ratio and/or the second output ratio is at most 0.05

In some embodiments, the method is carried out such that an ambient light component (for example, at one or more specified wavelengths - for example, the wavelength of the coherent light) of the detected light field signal prevailing at the first location is substantially equal to and/or correlated with an ambient light component of the detected light field signal prevailing at the second location.

In some embodiments, the method is carried out such that a slowly-varying component of the detected light field signal prevailing at the first location is substantially equal to and/or correlated with an slowly-varying component of the detected light field signal prevailing at the second location.

In some embodiments, the method is carried out such that a rapidly-varying non-stochastic rapidly-varying component of the detected light field signal prevailing at the first location is substantially equal to a non-stochastic rapidly-varying component of the detected light field signal prevailing at the second location.

In some embodiments, the method is carried out so that a ratio between an intensity, at a wavelength of the at least partially coherent light, of the detected light field signal prevailing at the first location and intensity , at a wavelength of the at least partially coherent light, of the detected light field signal prevailing at the second location is between 0.95 and 1.05.

## BRIEF DESCRIPTION OF THE DRAWINGS

FIG. 1 is an illustration of a DLS measurement based system for measuring one or more blood parameters (reproduced from WO 2008/053474).

FIG. 2 is an illustration of a system for generating an analog electrical signal *A(t)*.

FIGS. 3A-3B and 7A-7B are flow charts of routines for determining at least one blood parameter according to some embodiments.

FIG. 4 is a circuit diagram of an exemplary analog electronic assembly **270** in accordance with some embodiments.

FIG. 5 is a diagram of a system for measuring blood parameter(s) including a coherent light source and a plurality of photodetectors.

FIG. 6 is an illustration of an exemplary apparatus including a pneumatic tube deployed to a subject's finger.

FIG. 8 is an illustration of the "underside" or "finger-facing surface" of the housing.

FIG. 9 provides a "cross-section view" of the apparatus of FIG. 6.

FIG. 10 illustrates an optical fiber-related embodiment.

FIGS. 11A-11B illustrate an embodiment where more than one pair of photo detectors is provided.

## DETAILED DESCRIPTION OF EMBODIMENTS

The claims below will be better understood by referring to the present detailed description of example embodiments with reference to the figures. The description, embodiments and figures are not to be taken as limiting the scope of the claims. It should be understood that not every feature of the presently disclosed methods and apparatuses for handling error correction is necessary in every implementation. It should also be understood that throughout this disclosure, where a process or method is shown or described, the steps of the method may be performed in any order or simultaneously, unless it is clear from the context that one step depends on another being performed first. As used throughout this application, the word "may" is used in a permissive sense (i.e., meaning "having the potential to'), rather than the mandatory sense (i.e. meaning "must").

WO 2008/053474, incorporated herein by reference in its entirety, discloses a system and method for in vivo measurement of biological parameters.

In particular, WO 2008/053474 discloses a novel optical technique suitable for the in vivo measurement in a subject utilizing dynamic light scattering (DLS) approach. The effect of DLS are utilized for the measurement of variety of blood related parameters, such as viscosity of the blood and blood plasma, blood flow, arterial blood pressure and other blood chemistry and rheology related parameters such as concentration of analyte (e.g. glucose, hemoglobin, etc.), oxygen saturation etc.

DLS is a well-established technique to provide data on the size and shape of particles from temporal speckle analysis. When a coherent light beam (laser beam, for example) is incident on a scattering (rough) surface, a time-dependent fluctuation in the scattering property of the surface and thus in the scattering intensity (transmission and/or reflection) from the surface is observed. These fluctuations are due to the fact that the particles are undergoing Brownian or regular flow motion and so the distance between the particles is constantly changing with time. This scattered light then undergoes either constructive or destructive interference by the surrounding particles and within this intensity fluctuation information is contained about the time scale of movement of the particles. The scattered light is in the form of speckles pattern, being detected in the far diffraction zone. The laser speckle is an interference pattern produced by the light reflected or scattered from different parts of an illuminated surface. When an area is illuminated by laser light and is imaged onto a camera, a granular or speckle pattern is produced. If the scattered particles are moving, a time-varying speckle pattern is generated at each pixel in the image. The intensity variations of this pattern contain information about the scattered particles. The detected signal is amplified and digitized for further analysis by using the autocorrelation function (ACF) technique. The technique is applicable either by heterodyne or by a homodyne DLS setup.

The kinetics of optical manifestations of two kinds of physiological signals is measured *in vivo*: the pulsatile signal associated with heart beats and the post-occlusion optical signal which is induced by an artificially generated blood flow cessation. The light transmission and/or reflection signals are used as a control of the physiological response. This kind of control measurement can be carried out simultaneously with the DLS reflection measurement. The mutual correspondence between DLS and standard optical signals is subject to a comparison analysis.

Embodiments of the present invention relate to techniques for boosting a 'dynamic light scattering'-related and/or a stochastic and/or a 'coherent light interference pattern'-related component (i.e. relative to the overall signal) of analog signals obtain from photodetectors. Throughout the present disclosure, many teachings are described with respect to one specific case where light is scattered off of red blood cells in blood plasma that is circulating within the subject's blood vessels to effect an *in vivo* measurement.

This is not a limitation. Other embodiments may relate to *in vitro* measurement, measurement of particles within other biological fluids other than blood plasma (e.g. sweat, urine, lymph), or measurement of non-biological particles (e.g. colloids) within non-biological fluids (e.g. petroleum-based or any other non-biological fluid).

Definitions

For convenience, in the context of the description herein, various terms are presented here. To the extent that definitions are provided, explicitly or implicitly, here or elsewhere in this application, such definitions are understood to be consistent with the usage of the defined terms by those of skill in the pertinent art(s). Furthermore, such definitions are to be construed in the broadest possible sense consistent with such usage.

Analog electrical signals or light fields may comprises more than one sub-signal added together in a single electrical (or optical) signal. For example, an analog electrical signal derived from a light field detected by a photodetector that (i.e. where scattered light that is scattered from particles within a fluid contributed to the light field) may be the sum of: (i) a first component (i.e. analog electrical sub-signal) attributable to ambient light (e.g. sunlight); (ii) a second component attributable to skin light-modulating effects; (iii) a third component attributable to regular fluctuations in light intensity due to the presence of a fluorescent bulb and (iv) a fourth component attributable to scattered light that is scattered from particles within a fluid contributed to the light field. Each component or sub-signal of the analog electrical signal is associated with a different respective amount of power.

In some examples, for an analog signal generated by a photodetector, the relative power contribution to overall analog signal power attributable to ambient light is relatively high (i.e. the first component), while the relative power contribution to overall analog signal power attributable to scattered light that is scattered from particles within a fluid is relatively low (i.e. second component).

In general, both a signal and a sub-signal have power levels - the fraction of the power level of the overall signal attributable to a particular portion of the signal or sub-signal is the 'power fraction' of the sub-signal or signal component. In the example of the previous paragraph, the power fraction of the overall analog electrical signal due to the ambient light component may be significant (e.g at least 0.1 or at least 0.3 or at least 0.5) while the power fraction of the overall analog electrical signal due to the 'light scattering' component (i.e. fourth component) may be relatively low - for example, at most 0.1 or at most 0.05 or at most 0.01).

Embodiments of the present invention relate to generating a 'hybrid' signal. A 'hybrid signal' derived from a plurality of input analog signals is any non-zero or non-trivial mathematical combination of the input analog signals - i.e.. including multiplication, addition, subtraction, etc. The term 'hybrid' refers to the fact that the output (or hybrid) signal relates to more than one input signal, and is not restricted to a single input.

Embodiments of the present invention relate to photodetectors (any technology may be used including those listed herein or any other technology). In some embodiments, each photodetector is not infinitesimally small but rather has a size. The 'distance' between photodetectors relates to a centroid-centroid distance.

In some embodiments, a light field is comprised of more than on component. Whenever light is generated and reflected or scattered (or modulated in any other manner) to introduce photons into (or to pass through) a certain location (and/or to illuminate the location), this light 'contributes to' or 'influences' the local light field at that certain location.

Embodiments of the present invention relate to optically measuring a paremter relating to a subject. In different embodiments, this subject is human, or a mammal other than human, or to a warm-blooded animal other than mammals (e.g. birds).

Whenever a power level of a second signal is 'significantly less' than a power level of a first signal, a ratio between a power level of the second signal and a power level of the first signal is at most 0.5 or at most 0.3 or at most 0.2 or at most 0.1 or at most 0.05 or at most 0.01.

Some embodiments of the present invention are described for the specific case of only two photodetectors and/or measuring a light field in two locations. The skilled artisan will appreciate that this is not a limitation, any teaching disclosed herein may relate to the case of more than two photodetectors or detecting light fields in more than two locations. Thus, two photodetectors refers to 'at least two,' 'two locations' refers to at least two, and so on.

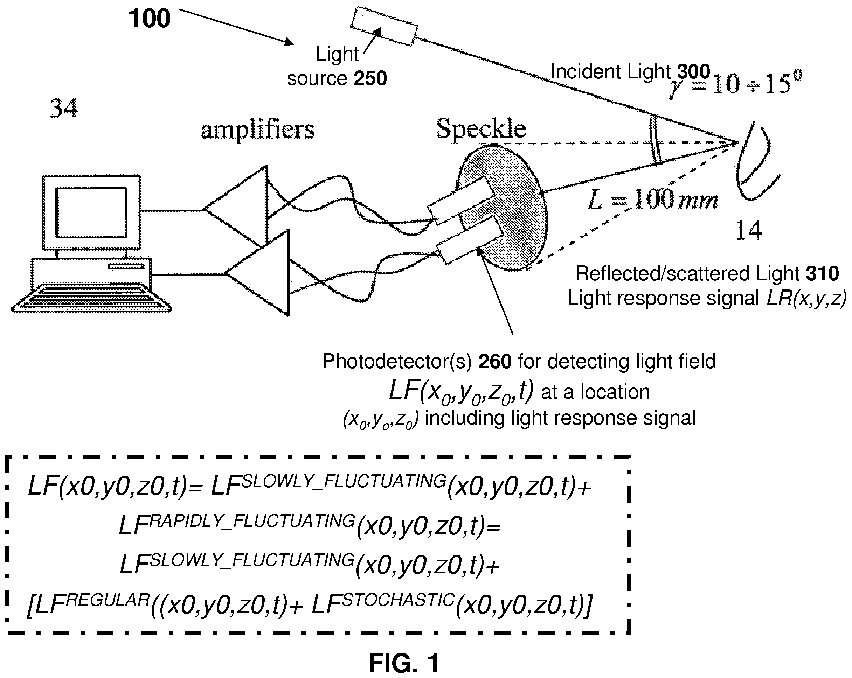

A Discussion of FIG. 1

FIG. 1, reproduced from WO 2008/053474, is an illustration of a DLS measurement based system for measuring one or more blood parameters. System 100 includes a light source unit **250** (e.g. laser) for generating at least partially coherent light; optical arrangement (not shown) including focusing optics and possibly also collecting optics; and a detection unit including a photodetector **260**. A focused beam of light **300** produced by laser **250** (e.g., a semiconductor laser) is used as a localized light source. In a non-limiting example, a light source unit **250** may be a laser diode (650 nm, 5mW) or VCSEL (vertical cavity surface emitting laser). The light response i.e. the reflected and/or transmitted light returned from the localized region of the subject's surface **14** (subject's finger in the present example) illuminated with the localized light source **250,** can be collected in a determined distance L (in a non-limiting example, L=100 mm) either directly by a detector or via multimode fiber optics. In a non-limiting example, the multimode fiber optics may be a bi-furcated randomized optical fiber where one optical entrance is connected to the detector and another one is optically coupled with the laser diode. In particular, as shown in Fig. 1, system **100** includes at least one laser diode and at least one photodetector (for example, photodiode(s)) appropriately positioned in the reflection-mode measurement set-up.

The photodetector **260** is positioned in space at location (x0,y0,z0) and is configured to detect a light field $LF(x_0,y_0,z_0)$ - i.e. the light field that exists/prevails at point $(x_0,y_0,z_0)$. Typically, the light detected by photodetector **260** comes from a number of sources including but not limited to (A) reflected light **310** which is reflected from and/or scattered by the biological tissue; and (ii) ambient light. Thus, it is possible to write:

$$LF(x_0, y_0, z_0) = LF_{reflected}(x_0, y_0, z_0) + LF_{ambient}(x_0, y_0, z_0) + \text{other term(s)}$$

(EQ. 1)

Throughout the present disclosure, $LF$ denotes a light field.

When light from light source **250** is incident upon biological tissue, (i) a first portion of the incident light is reflected from or scattered from "Brownian particles" (i.e. particles undergoing Brownian motion within a liquid - for example, red blood cells or thrombocytes) to generate a first light response signal whose magnitude/intensity varies stochastically and rapidly in time - this first light response signal is referred to as $LF_{reflected\_brownian}(x_0,y_0,z_0)$; (ii) a second portion of the incident light is reflected from stationary biological matter other than Brownian particles - for example, from skin cells, etc - this second portion of the incident light generates a second light response signal whose magnitude/intensity varies at most "slowly" and/or is not stochastic in time - this second light response signal is referred to as $LF_{reflected\_non\_brownian}(x_0,y_0,z_0)$;

Thus, it is possible to write:

$$LF_{reflected}(x_0, y_0, z_0) = LF_{reflected\_non\_brownian}(x_0, y_0, z_0) + LF_{reflected\_brownian}(x_0, y_0, z_0) + \text{other term(s)}$$

(EQ. 2)

In some embodiments, $LF_{reflected\_brownian}(x_0,y_0,z_0)$ is indicative of a dynamic light scattering parameter. Unfortunately, in many clinical situations $\dfrac{LF_{reflected\_brownian}(x_0, y_0, z_0)}{LF(x_0, y_0, z_0)}$ and/or $\dfrac{LF_{reflected\_brownian}(x_0, y_0, z_0)}{LF_{reflected\_non\_brownian}(x_0, y_0, z_0)}$ and/or

$$\frac{LF_{reflected\_brownian}(x_0, y_0, z_0)}{LF_{reflected\_ambient}(x_0, y_0, z_0)}$$ is "small" (for example, less than 0.1 or less than 0.01 or even smaller).

Embodiments of the present invention relate to apparatus and methods for "boosting" the relative contribution to an analog electrical signal of a component indicative of a dynamic light scattering measurement - for example, boosting the relative contribution of an analog electrical signal indicative of $LF_{reflected\_brownian}(x_0, y_0, z_0)$.

It is noted that, typically, $LF_{ambient}(x_0, y_0, z_0)$ (see Eqn. 1) and $LF_{reflected\_non\_brownian}(x_0, y_0, z_0)$ (see Eqn. 2) have an intensity that is either: (i) "slowly" fluctuating (for example, substantially constant or fluctuating at a rate less than 50 HZ); and/or (ii) "regularly behaved" and non-stochastic. One example of a "rapidly" fluctuating light signal that is regularly behaved and non-stochastic is light from a fluorescent light bulb operating at 50 HZ or 60 HZ. In contrast, the intensity of "speckles pattern light signal" $LF_{reflected\_brownian}(x_0, y_0, z_0)$ varies stochastically and rapidly - i.e. at a rate that is at least 50 HZ or at least 100 HZ or at least 200 HZ, depending on diffusion coefficient of the Brownian particle.

Thus, it is possible to write:

$$LF(x_0, y_0, z_0) = LF_{slowly\_fluctuating}(x_0, y_0, z_0) + \underbrace{\left[LF_{regular}(x_0, y_0, z_0) + LF_{stochastic}(x_0, y_0, z_0)\right]}_{rapidly-fluctuating} + \text{other terms} \quad (EQ. 3)$$

where (i) $LF_{slowly\_fluctuating}(x_0, y_0, z_0)$ is due to ambient light $LF_{ambient}(x_0, y_0, z_0)$ and/or light reflected from biological tissue other than Brownian particles $LF_{reflected\_non\_brownian}(x_0, y_0, z_0)$; (ii) rapidly-fluctuating (i.e. at a rate of greater than 50 HZ and/or 100 HZ and/or 200 HZ) $LF_{regular}(x_0, y_0, z_0)$ is due to ambient light $LF_{ambient}(x_0, y_0, z_0)$; and $LF_{stochastic}(x_0, y_0, z_0) = LF_{reflected\_brownian}(x_0, y_0, z_0)$/

For the present disclosure, "slowly fluctuating" refers to fluctuations at a rate of less than 50 HZ, while "rapidly fluctuating" refers to regular or stochastic fluctuations at a rate that exceeds 50 HZ (for example, at least 100 HZ or at least 200 HZ).

It is noted that: (i) $LF_{stochastic}(x_0, y_0, z_0)$ is the portion of $LF(x_0, y_0, z_0)$ that may be subjected to DLS analysis to yield one or more blood-related parameters; and (ii) in most clinical situations, $\frac{LF_{stochastic}(x_0, y_0, z_0)}{LF(x_0, y_0, z_0)}$ is relatively "small"

(for example, less than 0.1 or less than 0.01 or even smaller).

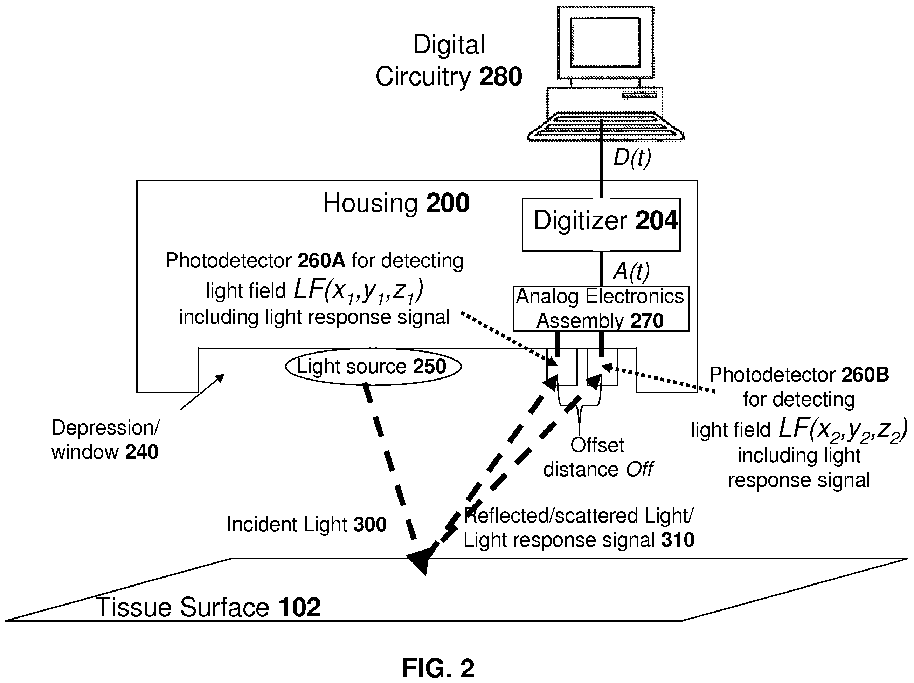

A First Discussion of a System Including Two Closely-Coupled Photodetectors With Reference to FIGS. 2-4

FIG. 2 is an illustration of a system for generating an analog electrical signal $A(t)$ that includes a relatively "large" component (for example, at least 0.01 or at least 0.1 or at least 0.2 or least 0.3 or at least 0.5 or least 0.8) that is indicative of a time-varying "speckles pattern light signal" received by one or more photo-detectors. This analog signal may be converted, using A to D converter or digitizer **204,** into a digital signal $D(t)$. The digital signal may be subjected to any analysis described in WO 2008/053474 by digital circuitry **280** to determine one or more blood parameters - for example, temporal autocorrelation or power spectrum techniques.

In a non-limiting example, the data is collected at 22 KHz sampling rate and 16-bit resolution, and then analyzed by digital circuitry **280.**

In the system of FIG. 2, light is received and detected by a plurality of photodetectors including: (i) photodetector **260A** for detecting the light field $LF(x_1, y_1, z_1)$ at location $(x_1, y_1, z_1)$; (ii) photodetector **260B** for detecting the light field $LF(x_2, y_2, z_2)$ at location $(x_2, y_2, z_2)$. Photodetector **260A** generates a first analog electrical signal $A_1(t)$ from $LF(x_1, y_1, z_1)$. Photodetector **260B** generates a second analog electrical signal $A_2(t)$ from $LF(x_2, y_2, z_2)$. Analog electronics assembly **270** receives the first $A_1(t)$ and second $A_2(t)$ analog electrical signals, and generates a "difference" between these two signals $A_1(t) - A_2(t)$ to produce analog electrical signal $A(t)$ which is digitized. Photodetectors **260B** and **260B** are positioned so that: (i) they are close enough together so that $LF_{ambient}(x_1, y_1, z_1)$ $LF_{ambient}(x_2, y_2, z_2)$, $LF_{reflected\_non\_brownian}(x_1, y_1, z_1)$ $\approx LF_{reflected\_non\_brownian}(x_2, y_2, z_2)$, $LF_{slowly\_fluctuating}(x_1, y_1, z_1) \approx LF_{slowly\_fluctuating}(x_2, y_2, z_2)$ and $LF_{regular}(x_1, y_1, z_1) \approx LF_{regular}(x_2, y_2, z_2)$; and (ii) they are far enough from each other such that the rapidly fluctuating $LF_{stochastic}(x_1, y_1, z_1)$ and $LF_{stochastic}(x_2, y_2, z_2)$ are not correlated with each other.

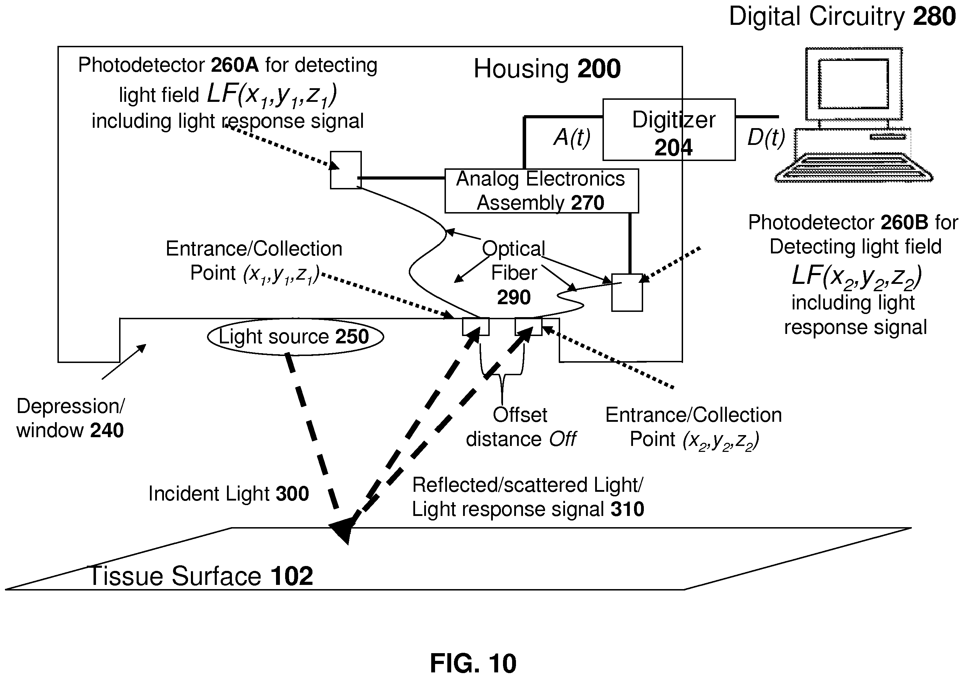

There is no limitation on any separation distance between $(x_1, y_1, z_1)$ and $(x_2, y_2, z_2)$. In some embodiments, in order for $LF_{stochastic}(x_1, y_1, z_1)$ and $LF_{stochastic}(x_2, y_2, z_2)$ to be uncorrelated, $(x_1, y_1, z_1)$ and $(x_2, y_2, z_2)$ should be separated by at least 0.01 mm or at least 0.05 mm or at least 0.1 or at least 0.2 mm or at least 0.3 mm or at least 0.5 mm or at least 1mm. Thus, in some embodiment, the offset distance $Off$ of FIG. 2 and 10 should be at least 0.01 mm or at least 0.05 mm or at least 0.1 or at least 0.2 mm or at least 0.3 mm or at least 0.5 mm or at least 1mm

In some embodiments, in order for $LF_{reflected\_non\_brownian}(x_1,y_1,z_1) \approx LF_{reflected-non\_brownian}(x_2,y_2,z_2)$, $LF_{slowly\_fluctuating}(x_1,y_1,z_1) \approx LF_{slowly\_fluctuating}(x_2,y_2,z_2)$ and $LF_{regular}(x_1,y_1,z_1) \approx LF_{regular}(x_2,y_2,z_2)$, then $(x_1,y_1,z_1)$ and $(x_2,y_2,z_2)$ should be separated by at most 10 cm or at most 5 cm or at most 3 cm or at most 2 cm or at most 1 cm or at most 0.5 mm or at most 0.25 or at most 1 mm.. Thus, in some embodiments, the offset distance *Off* of FIG. 2 and 10 should be at most 10 cm or at most 5 cm or at most 3 cm or at most 2 cm or at most 1 cm or at most 0.5 mm or at most 0.25 or at most 1 mm

In this case, if $A(t)=A_1(t)-A_2(t)$ represents $LF(x_1,y_1,z_1)-LF(x_2,y_2,z_2)$, then it is possible to write, using equation (3), that $A(t)$ represents

$$\left[LF_{slowly\_fluctuating}\left(x_1,y_1,z_1\right)-LF_{slowly\_fluctuating}\left(x_2,y_2,z_2\right)\right]+$$
$$\underbrace{\left\{\left[LF_{regular}\left(x_1,y_1,z_1\right)-LF_{regular}\left(x_2,y_2,z_2\right)\right]+\left[LF_{stochastic}\left(x_1,y_1,z_1\right)-LF_{stochastic}\left(x_2,y_2,z_2\right)\right]\right\}}_{rapidly-fluctuating}\cdot$$

Eq(4)

In the special case where (i) exact equality prevails - i.e. $LF_{slowly\_fluctuating}(x_1,y_1,z_1) = LF_{slowly\_fluctuating}(x_2,y_2,z_2)$, $LF_{regualar}(x_1,y_1,z_1) = LF_{regular}(x_2,y_2,z_2)$ and where (ii) rapidly fluctuating $LF_{stochastic}(x_1,y_1,z_1)$ and $LF_{stochastic}(x_2,y_2,z_2)$ are not correlated with each other, then $A(t)$ is a completely stochastic signal (i.e. indicative of a time-varying speckles pattern or DLS measurement produced by scattering from the Brownian particles), in contrast to $A_1(t)$ and $A_2(t)$ where the stochastic components of the signal may only be some fraction less than ½, for example, less than 0.1 or less than 0.01. Practically, $A(t)$ may also include some non-stochastic component. Nevertheless, in typical cases, the relative contribution of the non-stochastic component(s) (i.e., not due to scattering light on Brownian particles to generate a speckles pattern having a rapidly-varying intensity) to analog electric signal $A(t)$ is less than the contribution of respective non-stochastic components to $A_1(t)$ or $A_2(t)$.

A Discussion of a DLS Contribution Ratios

Consider the following ratios:

(i) a first DLS contribution ratio between (A) a magnitude of a "DLS component" of analog signal $A_1(t)$ (i.e. a component that is indicative of a DLS measurement - i.e. indicative of $LF_{stochastic}(x_1,y_1,z_1)$ or $LF_{Brownian}(x_1,y_1,z_1)$) and (B) $A_1(t)$, i.e.

$$\frac{\left|\text{analog electrical component of } A_1(t) \text{ indicative of } LF_{Brownian}\left(x_1,y_1,z_1\right)\right|}{\left|A_1(t)\right|} -$$

in many clinical situations, this first DLS ratio is at most 0.1 or at most 0.05 or at most 0.02 or at most 0.01, or even less.
(ii) a second DLS contribution ratio between (A) a magnitude of a "DLS component" of analog signal $A_2(t)$ (i.e. a component that is indicative of a DLS measurement - i.e. indicative of $LF_{stochastic}(x_2,y_2,z_2)$ or $LF_{Brownian}(x_2,y_2,z_2)$) and (B) $A_2(t)$, i.e.

$$\frac{\left|\text{analog electrical component of } A_2(t) \text{ indicative of } LF_{Brownian}\left(x_1,y_1,z_1\right)\right|}{\left|A_2(t)\right|} -$$

in many clinical situations, this second DLS ratio is at most 0.1 or at most 0.05 or at most 0.02 or at most 0.01, or even less.
(iii) a "boosted DLS contribution ratio" between (A) a magnitude of a "DLS component" of processed analog signal $A(t)$ (i.e. a component that is indicative of a DLS measurement(s) - i.e. indicative of a combination of (i) $LF_{stochastic}(x_1,y_1,z_1)$ or $LF_{Brownian}(x_1,y_1,z_1)$ and (ii) $LF_{stochastic}(x_2,y_2,z_2)$ or $LF_{Brownian}(x_2,y_2,z_2)$) and (B) $A_2(t)$, i.e.

$$\frac{\left|\text{analog electrical component of } A(t) \text{ indicative of a combination of } LF_{Brownian}(x_1, y_1, z_1) \text{ and } LF_{Brownian}(x_2, y_2, z_2)\right|}{\left|A_2(t)\right|}$$

in many clinical situations, this "boosted" DLS ratio exceeds the first and second DLS ratios is at least 0.05 or at least 0.1 or at least 0.2 or at least 0.5.

In some embodiments, a ratio between the "boosted DLS contribution ratio" and the "first DLS contribution ratio" and/or the "second DLS contribution ratio" is at least 3 or at least 5 or at least 10 or at least 20 or at least 50 or at least 100.

This "boosted ratio" may prevail for any length of time - for example, for at least 1 or 3 or 5 seconds,.

It is now disclosed that, in some embodiments, the greater this "ratio" between the "boosted DLS contribution ratio" and the first and/or second DLS contribution ratio, the easier it is to utilize processed analog signal $A(t)$ to determine one or more physiological parameters, as the "Brownian" component is greater. This may obviate the need for signal amplification and/or the need for using a digitizer with "many" channels, allowing the use of a digitizer with "fewer" channels.

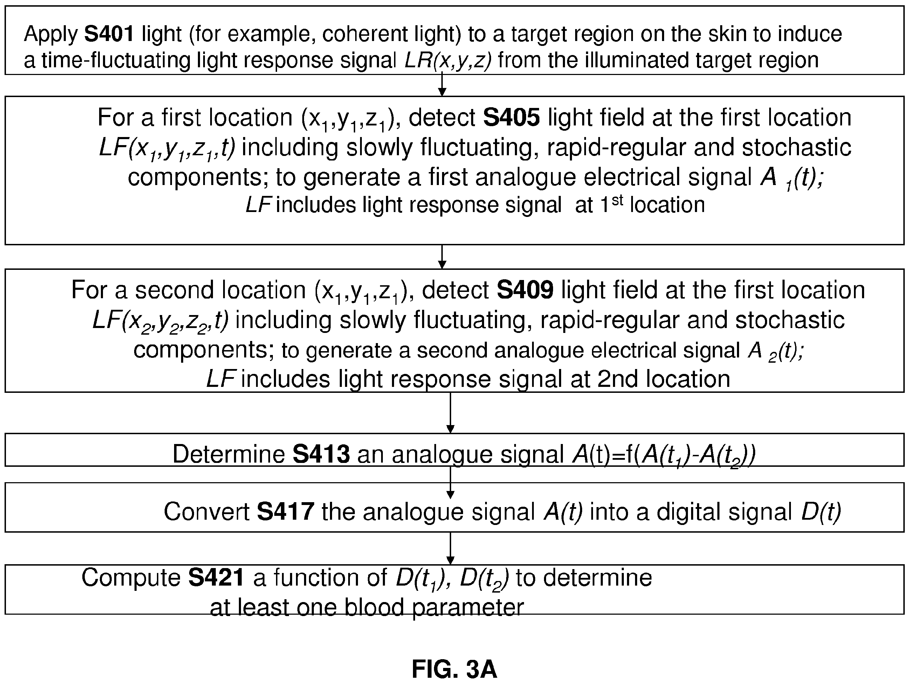

A Discussion of FIGS. 3A-3B

FIGS. 3A-3B are flow charts of routines for determining at least one blood parameter according to some embodiments. In step **S401,** light (for example, coherent light from light source **250**) is applied to a target region of the biological tissue to induce a time-fluctuating light response signal from the illuminated target region. In step **S405,** a light field $LF(x_1,y_1,z_1)$ prevailing at a first location $(x_1,y_1,z_1)$ is determined to generate a first $A_1(t)$ analog electrical signal. In step **S409,** a light field $LF(x_2,y_2,z_2)$ prevailing at a second location $(x_2,y_2,z_2)$ is determined to generate a second $A_2(t)$ analog electrical signal. In step **S413,** an analogue signal $A(t) = A_1(t) - A_2(t)$ is determined. In step **S417,** this analog signal is converted into a digital signal (for example, by digitizer **204**). In step **S421,** this digital signal is analyzed (for example, using any technique disclosed in WO 2008/053474 - for example, one or more of temporal autocorrelation or power spectrum techniques) to compute one or more blood parameters.

It is appreciate that the $A(t) = A_1(t) - A_2(t)$ refers to the simple case (and in many embodiments, this is preferred). However, it is possible to modify this technique of effecting an 'analog subtraction' where one or more of $A_1(t)$ or $A_2(t)$ is modified - for example, one or more of $A_1(t)$ or $A_2(t)$ is multiplied by a non-zero constant (or any other function) - for example, $A(t) = C1 * A_1(t) - C2 * A_2(t)$ (where $C1$ and $C2$ are approximately but not exactly equal - e.g. equal within a tolerance of at most 20% or at most 10% or at most 5% or at most 3% or at most 1% or at most 0.5% or at most 0.1%). In different embodiments, the magnitude of $A(t)$ is nevertheless much less than a magnitude of either $A_1(t)$ or $A_2(t)$ - thus, $A(t) = A_1(t) - A_2(t)$ substantially 'cancels' (without completely cancelling) the analog signal.

Furthermore, FIGS. 3A-3B are not limiting. In some embodiments, instead of deriving a blood parameter or physiological parameter (or any other parameter related to motion of light-scattering particles within a fluid) from a digitized signal, it is possible to analyze the 'difference' or 'hybrid' analog signal and/or to derive the blood parameter or physiological parameter (or any other parameter related to motion of light-scattering particles within a fluid) using analog circuitry.

For the present disclosure, deriving a 'motion-related' parameter and/or analyzing (or derivative thereof) may be carried out using any combination of analog circuitry, digital circuitry, or software (e.g. computer code executed by a computer processor such as a microprocessor).

Thus, the configuration in FIGS. 1-2 where there is a distinct 'division of work' between the analog and digital circuitry is one example, but is not intended as limiting.

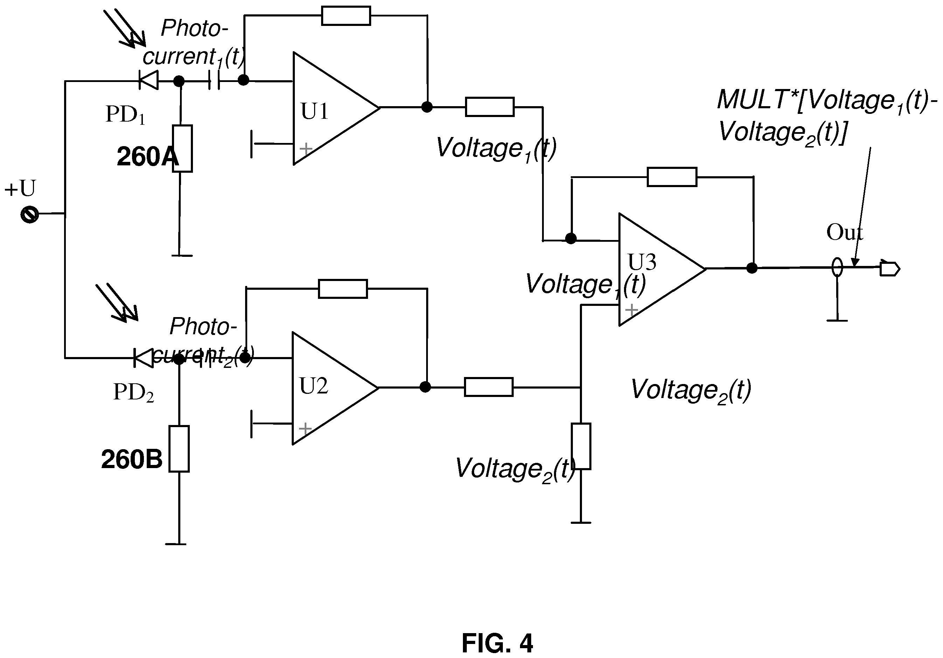

FIG. 4 is a circuit diagram of an exemplary analog electronic assembly **270** in accordance with some embodiments. Photocurrent $Photocurrent_1(t)$ - generated by the first photodetector **260A** is converted by a "first cascade" operational amplifier $U1$ from a "current analog signal" $Photocurrent_1(t)$ to a "voltage analog signal" $voltage_1(t)$-$Photocurrent_1(t)$ and $voltage_1(t)$ are non-limiting examples of "first analog signals" $A_1(t)$ discussed above with reference to FIGS.2-3.

Photocurrent $Photocurrent_2(t)$ - generated by the second photodetector **260B** is converted by a "first cascade" operational amplifier $U2$ from a "current analog signal" $Photocurrent_2(t)$ to a "voltage analog signal" $voltage_2(t)$-$Photocurrent_2(t)$ and $voltage_2(t)$ are non-limiting examples of "second analog signals" $A_2(t)$ discussed above with reference to FIGS.2-3.

"Second cascade" operational amplified $U3$ (i) receives as an input $voltage_1(t)$ and $voltage_2(t),$ and outputs a signal that is $MULT[voltage_1(t) - voltage_2(t)],$ which is the difference between $voltage_1(t)$ and $voltage_2(t)$ multiplied by a constant whose value is $MULT.$ It is noted that $MULT[voltage_1(t) - voltage_2(t)]$ is one example of $A(t)$ discussed above with reference to FIGS. 2-3.

The assembly of FIG. 4 is one example of a device that can generate a difference analog signal andor a 'hybrid' analog signal.

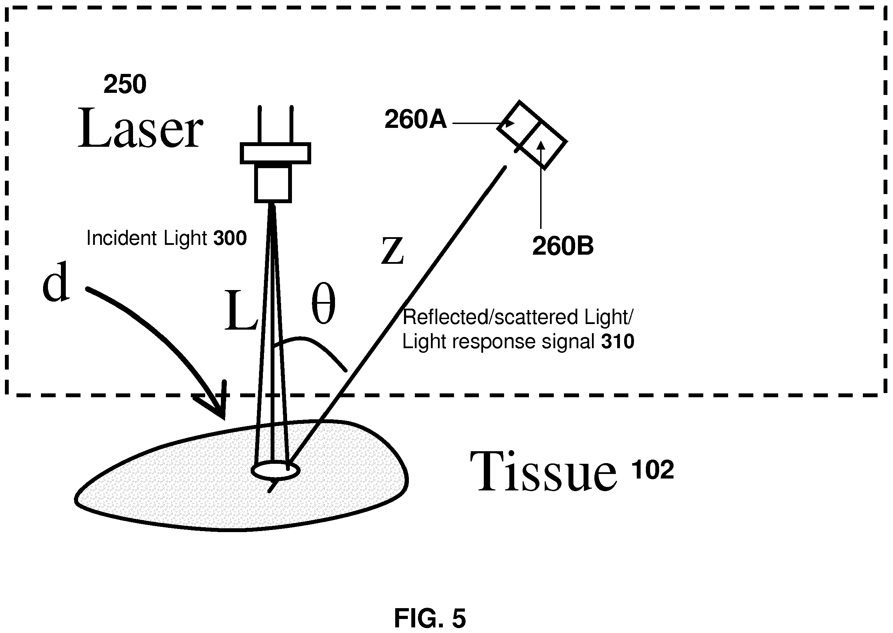

A Discussion of FIG. 5

FIG. 5 is a diagram of a system for measuring blood parameter(s) including a coherent light source and a plurality of photodetectors. Light from a light source **300** is incident upon tissue **102** to produce a reflected/scattered light/light response signal **310** which is detected by the photodetectors **260**.

In FIG. 5 the mean speckles size is:

$$\langle a \rangle = \frac{\lambda z}{d} \text{ (Eq 5)}$$

Where λ - wavelength of the light.
z- distance between photodiode and the measured object.
d- laser spot diameter.

Approximately the number of the speckles on the active area $S_p$ of the photodiode **260** is:

$$N = \frac{S_p d^2}{\lambda^2 z^2} \text{ (Eq 6)}$$

The mean current, generated by each speckle size element of the photo detector, is:

$$\langle I_S \rangle = \frac{\langle I_p \rangle \lambda^2 z^2}{S_p d^2} \text{ (Eq 7)}$$

The mean (DC) photodiode current $\langle I_p \rangle$ forming by N currents $\langle I_S \rangle$ in compliance with Kirchhoffs current law. Each speckle area is an independent realization of the same stochastic process, so fluctuating (AC) parts of these currents connected by relation:

$$\langle i_p^2 \rangle = \sum_1^N \langle i_S^2 \rangle \quad \text{and} \quad \langle i_p \rangle = \langle i_S \rangle \sqrt{N} \text{ (Eq 8)}$$

For one element it is possible to take advantage of Gaussian assumption, were satisfied $\langle i_S \rangle = \langle I_S \rangle$. By substituting (3) into (4) and by taking into account that $\langle I_p \rangle = \xi W S_p$, where W is a light's power flux, after simplification, it is possible to obtain:

$$\langle i_p \rangle = \frac{\xi W \lambda z}{d} S_p^{1/2} \text{ (Eq 9)}$$

That is, amplitude of the current fluctuation is proportionally to square root of detector surface. Note, that this relation valid in conditions, when the photodetector collects only a small part of scattered light. This relation can be used to optimize the signal to noise relation. The mean shot noise current from the photodetector is:

$$\langle i_0 \rangle = \sqrt{2Be\langle I_p \rangle} \text{ (Eq 10)}$$

Where B - signal bandwidth ($f_2$-0), normalized to 1 Hz. e - electron charge 1.6e-19C
Dividing (5) to (6), we get:

$$SNR = \frac{\lambda z}{d} \sqrt{\frac{\xi W}{2Be}} \text{ (Eq 11)}$$

So $SNR \neq f(S_p)$ and proportionally depend of the square root of the power flux on the photodetector.

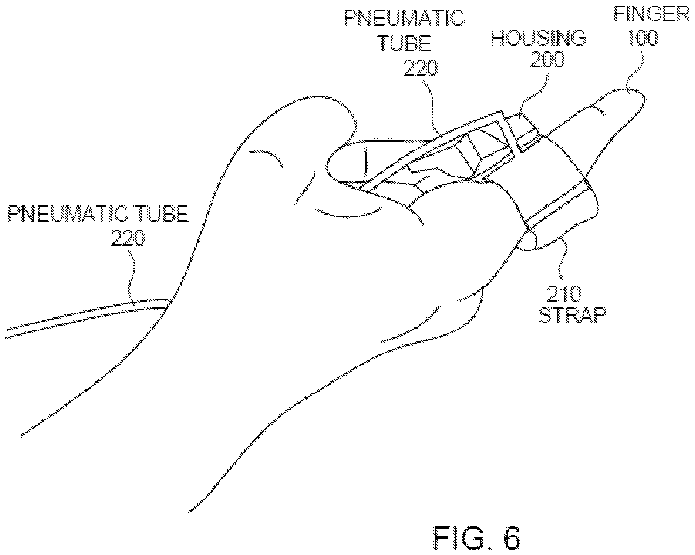

A Discussion of FIGS. 6-8: Embodiments Where a Force and/or Pressure is Applied to a Subject's Skin and/or Tissue to Induce a Change in Blood Flow

FIG. 6 is an illustration of an exemplary apparatus for: (i) inducing a change of blood flow in a subject's biological tissue (for example, in his/her finger or any other location on the subject's body); and (ii) generating an analog electric signal *A(t)* as discussed above. Towards this end, the apparatus of FIG. 6 includes a pneumatic tube **220** and a strap **210** that are mechanically coupled to a device housing **200**. Pneumatic tube **220** and strap **210** together form a pressurizing assembly that is operative to induce a change in blood flow. In the non-limiting example of FIG. 6, the pressurizing assembly including pneumatic tube **220** and strap **210** are operative to induce a change in blood flow in the subject's fingers. In yet another embodiment, pressurizing assembly includes some sort of vacuum assembly operative to provide a 'negative pressure' to a subject's back, and to induce a change in blood flow in the subject's back.

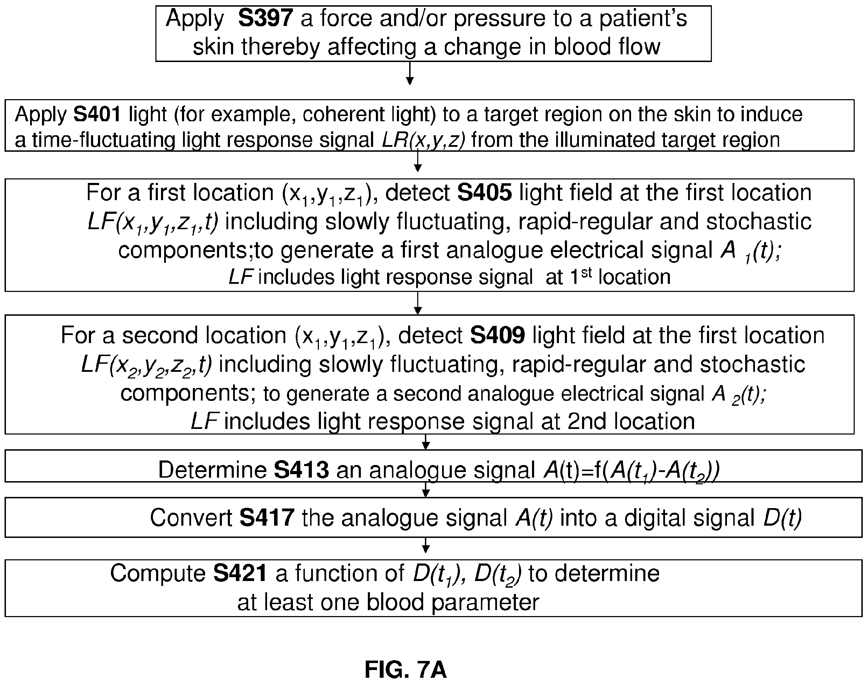

FIG. 7A is like FIG. 3A except there is an extra step of applying a force **S397** and/or pressure to a subject's biological tissue (for example, skin) to induce a change in blood flow. In some embodiments, carrying out this extra step before effecting the DLS measurement is useful for situations where it is desired to measure one or more of blood viscosity, blood particle size, and blood coagulation rate. Once again ,the skilled artisan is directed to WO 2008/053474.

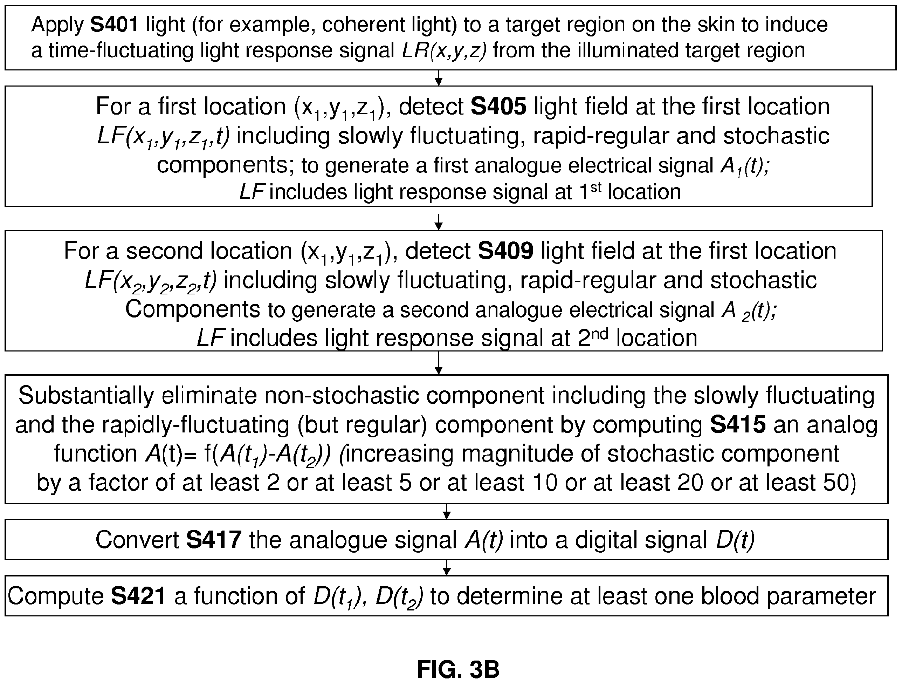

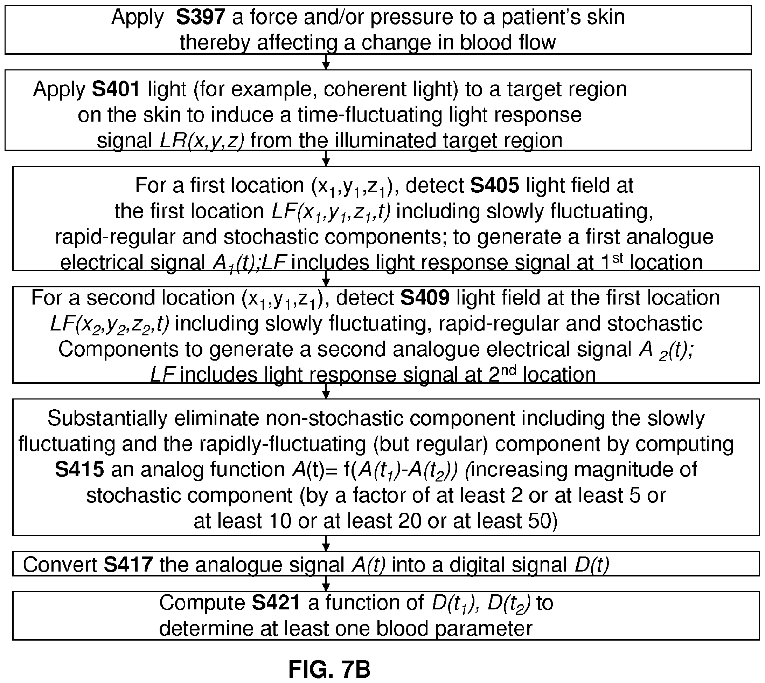

FIG. 7B is like FIG. 3B except there is an extra step of applying a force **S397** and/or pressure to a subject's biological tissue (for example, skin) to induce a change in blood flow.

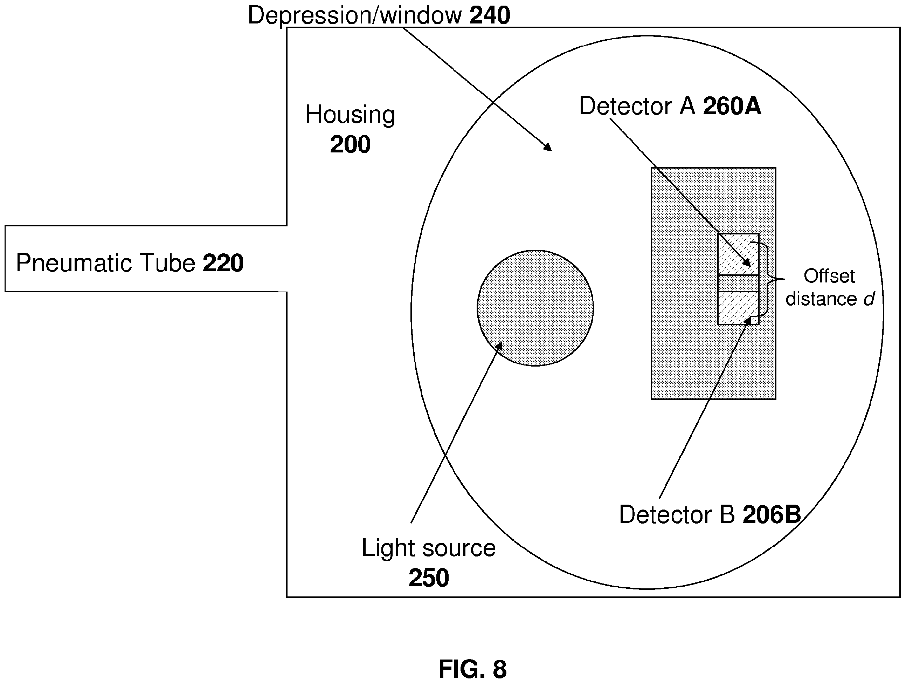

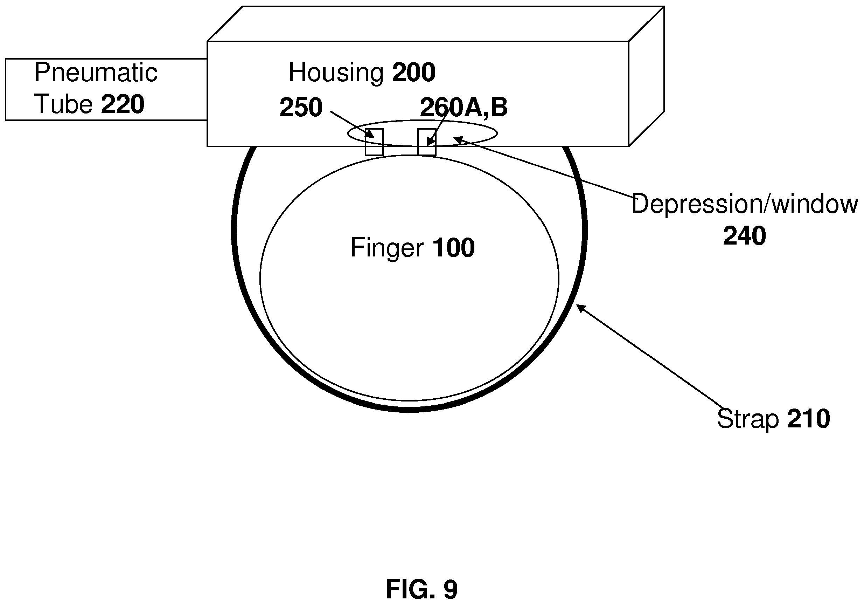

FIG. 8 is an illustration of the "underside" or "finger-facing surface" of housing **200**. On this "lower, finger-facing surface" of housing **200** there is a depression and/or window, within which light source **250,** and detectors **260A** and **260B** are situated. The skilled artisan is also directed to FIG. 9 which provides a "cross-section view" of the apparatus of FIG. 6.

A Discussion of FIG. 10 - Embodiments Related to Optical Fiber

In the non-limiting example of FIG. 2, photodetectors **260A** and **260B** are separated by an "Offset distance" *Off* whose value is selected as discussed above - thus, photodetector **260A** is located at position $(x_1,y_1,z_1)$ and photo-detector **260B** is located at position (x2,y2,z2).

This is not a limitation. Alternatively, as shown in FIG. 10, photodetector **260A** is located at a distance (for example, at least 1 or 3 or 5 or 10 cm) from $(x_1,y_1,z_1)$ and receives the optical signal of the light field prevailing at $(x_1,y_1,z_1)$ via optical fiber **290** and/or photodetector **260B** is located at a distance (for example, at least 1 or 3 or 5 or 10 cm) from $(x_2,y_2,z_2)$ and receives the optical signal of the light field prevailing at $(x_2,y_2,z_2)$ via another optical fiber **290.**

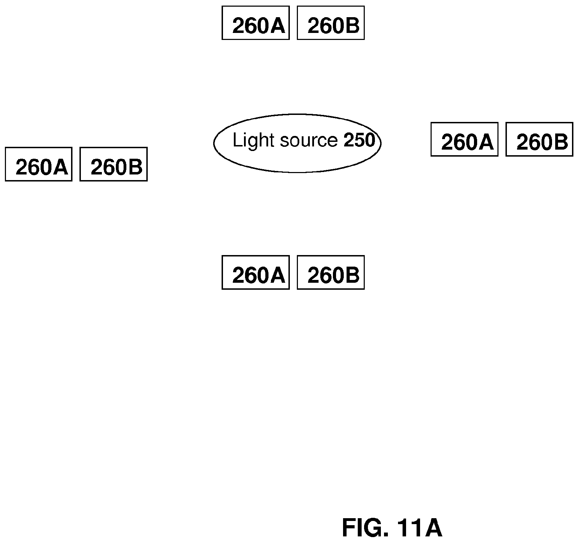

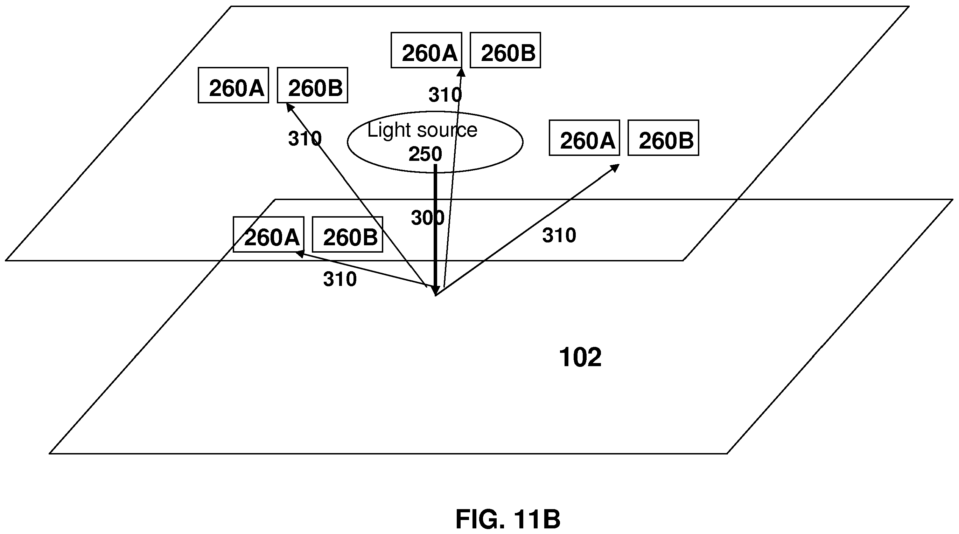

In some embodiments, as shown in FIGS. 11A-11B, more than one pair of photo detectors may be provided, and some sort of averaging between results associated with each pair of photodetectors may be computed, in order to determine a more accurate assessment of the subject's physiological parameter(s).

First Additional Discussion

In some embodiments, the behavior of a DLS related parameter *(d(ln(G)ldt))* utilized for the determination of systolic and diastolic blood pressure, where correlation function $G(\tau)$ of temporal intensity fluctuations of light scattered by moving particles is given by:

$$G(\tau) = \frac{< I(t) \cdot I(t+\tau) >}{< I(t) >^2}$$

. For example, there is a pressurizing cuff (for example, including pneumatic tube **210** and strap **220)** is inflated up to over systolic pressure of 200 mm Hg during the first 5 seconds. Thereafter, for the next 75 seconds, the air pressure in the cuff is gradually reduced. Simultaneously, the DLS measurement is carried out at the area beneath the cuff. In this case, the parameter *d(Ln(G))ldt* reaches its minimum point when the pressure measured in the cuff gets equal to the systolic pressure, as was defined previously by doing a standard blood pressure measurement test. Moreover, at the moment where the pressure in the cuff exceeds previously defined systolic pressure point, exactly at this point the value of parameter *d(Ln(G))/dt* starts to increase gradually. Therefore, by identifying these two extreme points on the curve of *d(Ln(G))/dt,* both systolic and diastolic blood pressure can be measured optically. Naturally, all other functions mathematically related to autocorrelation parameters, can be used for blood pressure measurement.

This very unique sensitivity of DLS related parameters to the blood flow can be used for identification of blood flow disturbances or even for blood stasis identification and verification. To this end, any kind of a medical tool such as intro-vascular catheter (e. g. used for angioplasty) can be linked with DLS equipped optical fiber. Such a system is very efficient for identification of plugs and blood vessels abnormalities disturbing the normal blood flow.

Moreover, blood circulation parameters measured by DLS technique can by embedded as an inherent part of new emerging technology of biofeedback. Based upon the biofeedback technique, different body parameters including the blood flow that can be beneficial to control emotional status, cardio-vascular training, rehabilitation and other purposes can be controlled. For example, such a system can be used for the control of blood flow during recovery from heart failure. In the biofeedback applications, DLS based measurement system can be combined with facilities affecting the mental status of a subject. For example, a method of binaural beats can be used. The binaural beats are resulted from the interaction of two different auditory impulses, originating in opposite ears. The binaural beat is not heard but is perceived as an auditory beat and theoretically can be used to entrain specific neural rhythms through the frequency-following response (FFR), *i.e.* the tendency for cortical potentials to entrain to or resonate at the frequency of an external stimulus. Thus, a consciousness management technique can be utilized to entrain a specific induction of sympathetic and parasympathetic system. More specifically, biofeedback system based on the methods of binaural beats can be governed by the parameters of flowing blood measured by means of DLS.

Second Additional Discussion

It is noted that in some embodiments related to FIG. 2 or other figures, photodetectors **260A** and **260B** may be (i) attached to housing **200** and/or (ii) integrally formed with housing **200** and/or (iii) rigidly maintained at a fixed distance from each other (for example, using housing **200).**

Digital circuitry **280** or digital processing unit **280** may include any software/computer readable code module and/or firmware and/or hardware element(s) including but not limited to a CPU, volatile or non-volatile memory, field programmable logic array (FPLA) element(s), hard-wired logic element(s), field programmable gate array (FPGA) element(s), and application-specific integrated circuit (ASIC) element(s). Any instruction set architecture may be used in digital circuitry **280** including but not limited to reduced instruction set computer (RISC) architecture and/or complex instruction set computer (CISC) architecture.

In some embodiments, any feature or combination of features WO 2008/053474 (for example, detecting any physiological parameter disclosed in WO 2008/053474 or implanting any computational technique disclosed in WO 2008/053474 or any other feature disclosed in WO 2008/053474) may be provided. Citation of a reference does not constitute an admission that the reference is prior art.

It is further noted that any of the embodiments described above may further include receiving, sending or storing instructions and/or data that implement the operations described above in conjunction with the figures upon a computer readable medium. Generally speaking, a computer readable medium may include storage media or memory media such as magnetic or flash or optical media, e.g. disk or CD-ROM, volatile or non-volatile media such as RAM, ROM, etc. as well as transmission media or signals such as electrical, electromagnetic or digital signals conveyed via a communication medium such as network and/or wireless links.

Having thus described the foregoing exemplary embodiments it will be apparent to those skilled in the art that various equivalents, alterations, modifications, and improvements thereof are possible without departing from the scope of the claims as hereafter recited. In particular, different embodiments may include combinations of features other than those described herein. Accordingly, the claims are not limited to the foregoing discussion.

**Claims**

**1.** A method for obtaining physiological information about a subject, the method comprising:

    a) illuminating a portion of the subject's skin by a laser diode or a vertical cavity surface emitting laser to scatter partially or entirely coherent visible or near infrared light off of moving red blood cells within the subject's blood to influence a location-dependent light field;
    b) detecting light of the location-dependent light field at first and second locations to respectively generate first and second analog electrical signals that respectively describe the light field at the first and second locations;

    **characterised in that** the method further comprises:

c) generating a difference analog electrical signal that describes a difference between the first and second analog signals;

d) analyzing the difference analog electrical signal or a derivative thereof to characterize time fluctuations of a coherent light interference pattern described by the difference analog signal; and

e) deriving, according to results of the analysis, at least one physiological parameter of the subject, the physiological parameter being selected from the group consisting of (i) a diffusion coefficient of a biological particle within the subject's blood and (ii) a blood coagulation rate.

2. The method of claim 1 wherein the derived physiological parameter of the subject is the diffusion coefficient of a biological particle within the subject's blood.

3. The method of claim 1 wherein the derived physiological parameter of the subject is the diffusion coefficient of a red blood cells within the subject's blood.

4. The method of claim 1 wherein the derived physiological parameter of the subject is the blood coagulation rate.

**Patentansprüche**

1. Verfahren zum Erhalten von physiologischer Information über eine Versuchsperson, wobei das Verfahren Folgendes umfasst:

a) Beleuchten eines Teils der Haut der Versuchsperson durch eine Laserdiode oder einen oberflächenemittierenden Laser mit vertikalem Resonator, um teilweise oder gänzlich kohärent sichtbares oder Nahinfrarot-Licht an beweglichen roten Blutzellen innerhalb des Blutes der Versuchsperson zu zerstreuen, um eine positionsabhängiges Lichtfeld zu beeinflussen;

b) Erkennen von Licht des positionsabhängigen Lichtfeld an ersten und zweiten Positionen, um jeweils erste und zweite analoge elektrische Signale zu erzeugen, die jeweils das Lichtfeld an den ersten und zweiten Positionen beschreiben;

**dadurch gekennzeichnet, dass** das Verfahren ferner Folgendes umfasst:

c) Erzeugen eines analogen elektrischen Differenzsignals, das eine Differenz zwischen den ersten und zweiten Analogsignalen beschreibt;

d) Analysieren des analogen elektrischen Differenzsignals oder einer Ableitung davon, um Zeitschwankungen eines kohärenten, durch das analoge Differenzsignal beschriebene Lichtinterferenzmuster zu charakterisieren; und

e) Ableiten, entsprechend der Analyseergebnisse, von mindestens einem physiologischen Parameter der Versuchsperson, wobei der physiologische Parameter aus der Gruppe bestehend aus (i) einem Diffusionskoeffizienten eines biologischen Teilchens innerhalb des Blutes der Versuchsperson und (ii) eine Blutkoagulationsgeschwindigkeit ausgewählt wird.

2. Verfahren nach Anspruch 1, wobei der abgeleitete physiologische Parameter der Versuchsperson der Diffusionskoeffizient eines biologischen Teilchens innerhalb des Blutes der Versuchsperson ist.

3. Verfahren nach Anspruch 1, wobei der abgeleitete physiologische Parameter der Versuchsperson der Diffusionskoeffizient von roten Blutzellen innerhalb des Blutes der Versuchsperson ist.

4. Verfahren nach Anspruch 1, wobei der abgeleitete physiologische Parameter der Versuchsperson die Blutkoagulationsgeschwindigkeit ist.

**Revendications**

1. Procédé permettant d'obtenir des informations physiologiques concernant un sujet, ledit procédé comprenant :

a) l'illumination d'une partie de la peau du sujet au moyen d'une diode laser ou d'un laser à cavité verticale émettant par la surface pour disperser partiellement ou entièrement une lumière cohérente visible ou proche infrarouge des globules rouges en déplacement dans le sang du sujet afin d'influencer un champ lumineux dépendant de l'emplacement ;

b) la détection de lumière du champ lumineux dépendant de l'emplacement aux premier et second emplacements pour générer respectivement des premiers et second signaux électriques analogiques qui décrivent respectivement le champ lumineux aux premier et second emplacements ;

**caractérisé en ce que** le procédé comprend en outre :

c) la génération d'un signal électrique analogique différentiel décrivant une différence entre les premier et second signaux analogiques ;

d) l'analyse du signal électrique analogique différentiel ou d'un dérivé de celui-ci afin de caractériser les variations temporelles d'un motif d'interférence de lumière cohérente décrit par le signal analogique différentiel ; et

e) la déduction, selon les résultats de l'analyse, d'au moins un paramètre physiologique du sujet, le paramètre physiologique étant choisi dans le groupe constitué par (i) un coefficient de diffusion d'une particule biologique dans le sang du sujet et (ii) une vitesse de coagulation du sang.

2. Procédé selon la revendication 1, ledit paramètre physiologique déduit du sujet étant le coefficient de diffusion d'une particule biologique dans le sang du sujet.

3. Procédé selon la revendication 1, ledit paramètre physiologique déduit du sujet étant le coefficient de diffusion des globules rouges dans le sang du sujet.

4. Procédé selon la revendication 1, ledit paramètre physiologique déduit du sujet étant la vitesse de coagulation du sang.

Photodetector(s) **260** for detecting light field
$LF(x_0, y_0, z_0, t)$ at a location
$(x_0, y_0, z_0)$ including light response signal

$$LF(x0,y0,z0,t) = LF^{SLOWLY\_FLUCTUATING}(x0,y0,z0,t) +$$
$$LF^{RAPIDLY\_FLUCTUATING}(x0,y0,z0,t) =$$
$$LF^{SLOWLY\_FLUCTUATING}(x0,y0,z0,t) +$$
$$[LF^{REGULAR}((x0,y0,z0,t) + LF^{STOCHASTIC}(x0,y0,z0,t)]$$

**FIG. 1**

EP 2 637 559 B1

Digital
Circuitry **280**

*D(t)*

Housing **200**

Digitizer **204**

Photodetector **260A** for detecting
light field $LF(x_1, y_1, z_1)$
including light response signal

*A(t)*

Analog Electronics
Assembly **270**

Light source **250**

Photodetector **260B**
for detecting
light field $LF(x_2, y_2, z_2)$
including light
response signal

Depression/
window **240**

Offset
distance *Off*

Incident Light **300**

Reflected/scattered Light/
Light response signal **310**

Tissue Surface **102**

**FIG. 2**

EP 2 637 559 B1

Apply **S401** light (for example, coherent light) to a target region on the skin to induce a time-fluctuating light response signal *LR(x,y,z)* from the illuminated target region

For a first location $(x_1,y_1,z_1)$, detect **S405** light field at the first location *LF(x₁,y₁,z₁,t)* including slowly fluctuating, rapid-regular and stochastic components; to generate a first analogue electrical signal $A_1(t)$; *LF* includes light response signal at 1ˢᵗ location

For a second location $(x_1,y_1,z_1)$, detect **S409** light field at the first location *LF(x₂,y₂,z₂,t)* including slowly fluctuating, rapid-regular and stochastic components; to generate a second analogue electrical signal $A_2(t)$; *LF* includes light response signal at 2nd location

Determine **S413** an analogue signal $A(t)=f(A(t_1)-A(t_2))$

Convert **S417** the analogue signal *A(t)* into a digital signal *D(t)*

Compute **S421** a function of $D(t_1)$, $D(t_2)$ to determine at least one blood parameter

**FIG. 3A**

EP 2 637 559 B1

Apply **S401** light (for example, coherent light) to a target region on the skin to induce a time-fluctuating light response signal *LR(x,y,z)* from the illuminated target region

For a first location $(x_1,y_1,z_1)$, detect **S405** light field at the first location $LF(x_1,y_1,z_1,t)$ including slowly fluctuating, rapid-regular and stochastic components; to generate a first analogue electrical signal $A_1(t)$; *LF* includes light response signal at 1st location

For a second location $(x_1,y_1,z_1)$, detect **S409** light field at the first location $LF(x_2,y_2,z_2,t)$ including slowly fluctuating, rapid-regular and stochastic Components to generate a second analogue electrical signal $A_2(t)$; *LF* includes light response signal at 2nd location

Substantially eliminate non-stochastic component including the slowly fluctuating and the rapidly-fluctuating (but regular) component by computing **S415** an analog function $A(t)= f(A(t_1)-A(t_2))$ (increasing magnitude of stochastic component by a factor of at least 2 or at least 5 or at least 10 or at least 20 or at least 50)

Convert **S417** the analogue signal *A(t)* into a digital signal *D(t)*

Compute **S421** a function of $D(t_1)$, $D(t_2)$ to determine at least one blood parameter

**FIG. 3B**

EP 2 637 559 B1

MULT*[Voltage$_1$(t)-Voltage$_2$(t)]

Out

Voltage$_1$(t)

U3

Voltage$_1$(t)

Voltage$_2$(t)

Voltage$_2$(t)

Voltage$_1$(t)

U1

U2

Photo-current$_1$(t)

Photo-current$_2$(t)

PD$_1$

PD$_2$

260A

260B

+U

FIG. 4

**250**

Laser

**260A**

Incident Light **300**

Z

**260B**

d

L θ

Reflected/scattered Light/
Light response signal **310**

Tissue **102**

**FIG. 5**

EP 2 637 559 B1

PNEUMATIC
TUBE
220

HOUSING
200

FINGER
100

PNEUMATIC TUBE
220

210
STRAP

FIG. 6

EP 2 637 559 B1

Apply **S397** a force and/or pressure to a patient's
skin thereby affecting a change in blood flow

Apply **S401** light (for example, coherent light) to a target region on the skin to induce
a time-fluctuating light response signal $LR(x,y,z)$ from the illuminated target region

For a first location ($x_1,y_1,z_1$), detect **S405** light field at the first location
$LF(x_1,y_1,z_1,t)$ including slowly fluctuating, rapid-regular and stochastic
components;to generate a first analogue electrical signal $A_1(t)$;
$LF$ includes light response signal at $1^{st}$ location

For a second location ($x_1,y_1,z_1$), detect **S409** light field at the first location
$LF(x_2,y_2,z_2,t)$ including slowly fluctuating, rapid-regular and stochastic
components; to generate a second analogue electrical signal $A_2(t)$;
$LF$ includes light response signal at 2nd location

Determine **S413** an analogue signal $A(t)=f(A(t_1)-A(t_2))$

Convert **S417** the analogue signal $A(t)$ into a digital signal $D(t)$

Compute **S421** a function of $D(t_1)$, $D(t_2)$ to determine
at least one blood parameter

**FIG. 7A**

Apply **S397** a force and/or pressure to a patient's skin
thereby affecting a change in blood flow

↓

Apply **S401** light (for example, coherent light) to a target region
on the skin to induce a time-fluctuating light response
signal *LR(x,y,z)* from the illuminated target region

↓

For a first location $(x_1,y_1,z_1)$, detect **S405** light field at
the first location *LF($x_1,y_1,z_1,t$)* including slowly fluctuating,
rapid-regular and stochastic components; to generate a first analogue
electrical signal $A_1(t)$;*LF* includes light response signal at 1st location

↓

For a second location $(x_1,y_1,z_1)$, detect **S409** light field at the first location
*LF($x_2,y_2,z_2,t$)* including slowly fluctuating, rapid-regular and stochastic
Components to generate a second analogue electrical signal $A_2(t)$;
*LF* includes light response signal at 2nd location

↓

Substantially eliminate non-stochastic component including the slowly
fluctuating and the rapidly-fluctuating (but regular) component by computing
**S415** an analog function $A(t)= f(A(t_1)-A(t_2))$ *(increasing magnitude of
stochastic component (by a factor of at least 2 or at least 5 or
at least 10 or at least 20 or at least 50)*

↓

Convert **S417** the analogue signal *A(t)* into a digital signal *D(t)*

↓

Compute **S421** a function of *D($t_1$)*, *D($t_2$)* to
determine at least one blood parameter

## FIG. 7B

**FIG. 8**

EP 2 637 559 B1

Pneumatic Tube **220**

Housing **200**

**250**    **260A,B**

Depression/window **240**

Finger **100**

Strap **210**

**FIG. 9**

**Digital Circuitry 280**

Photodetector **260A** for detecting light field $LF(x_1,y_1,z_1)$ including light response signal

Housing **200**

Digitizer **204**

$A(t)$

$D(t)$

Analog Electronics Assembly **270**

Entrance/Collection Point $(x_1,y_1,z_1)$

Optical Fiber **290**

Light source **250**

Photodetector **260B** for Detecting light field $LF(x_2,y_2,z_2)$ including light response signal

Depression/ window **240**

Offset distance *Off*

Entrance/Collection Point $(x_2,y_2,z_2)$

Incident Light **300**

Reflected/scattered Light/ Light response signal **310**

Tissue Surface **102**

**FIG. 10**

EP 2 637 559 B1

260A 260B

260A 260B

Light source 250

260A 260B

260A 260B

FIG. 11A

EP 2 637 559 B1

FIG. 11B

260A 260B

260A 260B

Light source
250

260A 260B

310

310

300

260A 260B

310

310

102

EP 2 637 559 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007144880 A
- WO 2007113804 A
- WO 2008053474 A
- US 4476865 A
- WO 2008053473 A2

### Non-patent literature cited in the description

- **MACFADYEN, A. J. ; B. R. JENNINGS.** Fibre-optic systems for dynamic light scattering-a review. *Optics & Laser Technology,* 1990, vol. 22.3, 175-187
- **KALCHENKO, VYACHESLAV et al.** In vivo dynamic light scattering imaging of blood coagulation. *Journal of biomedical optics,* 2007, vol. 12.5, 052002-052002